# EUROPEAN PATENT SPECIFICATION

(11) **EP 3 932 481 B1**
(45) Date of publication and mention of the grant of the patent: **27.12.2023**
(21) Application number: 20183082.5
(22) Date of filing: 30.06.2020
(51) Int. Cl.: A61N 5/10

(54) **MULTIMODAL PROTON THERAPY TREATMENT PLANNING SYSTEM**
BEHANDLUNGSPLANUNGSSYSTEM FÜR MULTIMODALE PROTONENTHERAPIE
SYSTÈME DE PLANIFICATION DE TRAITEMENT DE THÉRAPIE PROTONIQUE MULTIMODAL

(43) Date of publication of application: 05.01.2022
(73) Proprietor: Ion Beam Applications, 1348 Louvain-la-Neuve (BE)
(72) Inventor: LABARBE, Rudi, 1348 Louvain-la-Neuve (BE); HOTOIU, Lucian, 1348 Louvain-la-Neuve (BE); PIN, Arnaud, 1348 Louvain-la-Neuve (BE)
(74) Representative: Connor, Marco Tom

(56) References cited:
- EP-A1- 3 424 560
- WO-A1-2011/160235
- WO-A1-2017/093469
- WO-A1-2019/018813
- US-A1- 2005 207 531
- US-A1- 2012 136 194

## Description

### FIELD OF THE INVENTION

The present invention relates to a treatment planning system (TPS) for generating a plan for treatment with charged particles beams, preferably with proton beams, of a target tissue comprising tumoural cells. The plan fulfils predefined clinical criteria for ensuring that at the end of the treatment, the tumoural cells are destroyed or killed, whilst preserving the healthy cells adjacent to the tumoural cells. The TPS establishes the plan by combining irradiation sessions or fractions at conventional dose deposition rates (CDR) and / or at ultra-high dose deposition rates (HDR) according to criteria specific to the present invention. The plan thus generated ensures an effective treatment of the tumoural cells, with a lower ratio of damages to healthy cells adjacent to the tumoural cells.

### BACKGROUND OF THE INVENTION

Radiation therapy with particles or waves, such as electron beams, protons beams, heavy ions beams, x-rays, *γ*-rays, and the like, has become an essential tool for treating patients with tumours. Since both tumoural cells and healthy cells are damaged by such radiations, a major challenge in cancer treatment is to define a plan ensuring that the tumoural cells are effectively destroyed or killed, while sparing as much as possible the healthy cells, in particular those adjacent to the tumoural cells. Such plan must satisfy multiple, often competing, parameters, and is therefore quite complex. For this reason, treatment planning systems are generally computer generated.

A first criterion a treatment plan must fulfil is to ensure that, at the end of the treatment, a total target dose (DmtT) greater than or equal to a minimum target dose (DmtT0) has been delivered to the tumoural cells forming the target effective for destroying / killing the tumoural cells.

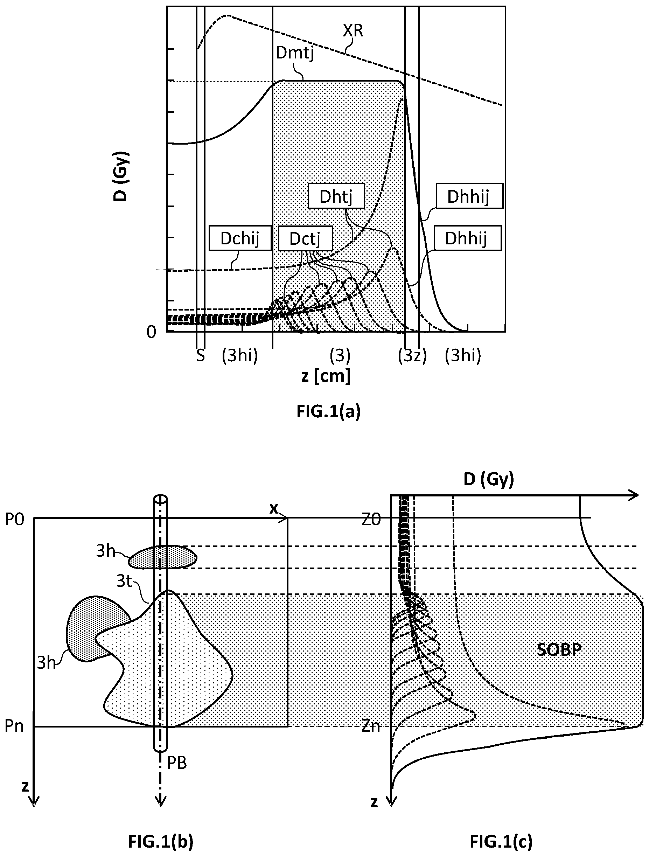

A second criterion to be fulfilled by the treatment plan is to minimize the degradation of the healthy cells adjacent to the tumoural cells. No matter how accurate is the dose deposition in a volume comprising tumoural cells, a radiation beam reaching the volume comprising the tumoural cells almost necessarily crosses healthy cells and delivers thereto a dose also to healthy cells surrounding or included in that volume. Different radiations deposit their energies in different patterns. For example, as shown in Figure 1, X-rays deposit most of their energy near the level of the skin, and the deposited energy decreases with depth. Healthy tissues located upstream of a target volume of tumoural cells therefore receive a higher dose than the tumoural cells of target volume. By contrast, protons deposit most of their energy close to the end of their beam path, forming a so-called Bragg peak. By superposing a number of beamlets with their respective Bragg peaks staggered in depth, a sum of individual Bragg Peaks (SOBP) can be defined spanning a whole depth of the volume of tumoural cells or of a fraction of that volume. The healthy cells located upstream of the volume of tumoural cells crossed by a proton beam therefore receive a lower dose than the tumoural cells in the volume. Consequently, proton therapy is well suited for depositing high doses in deep seated tumours.

To minimize the degradation of the healthy cells adjacent to the tumoural cells, the total dose (DhTi) received by a healthy cell (i) must not exceed a maximum allowable dose. As the maximum allowable dose a healthy cell can (relatively) safely receive in one session may be substantially lower than the minimum target dose (DmtT0) required to destroy the tumoural cells, the total target dose (DmtT) can be delivered to the tumoural cells in one or more fractions (or sessions).

A treatment plan can therefore include the delivery of a predefined total target dose (DmtT) of radiation to all the tumoural cells in N sessions or fractions j, with N ≥ 1, each fraction j delivering a target fraction dose (Dmtj) to a volume of tumoral cells. If N = 1, the whole of the predefined total target dose is delivered in a single session. If N > 1, the predefined total target dose is delivered in several fractions, possibly to different volumes of tumoral cells, such that at the end of the N^{th} session, all the tumoral cells have received a cumulated target dose equal to the predefined total target dose (DmtT) required for killing all the tumoural cells. The number N of fractions depends on the nature of the healthy tissues which determines the maximum fraction dose they can safely receive in a fraction. It also depends on the type of radiation used and on the positions of the healthy cells relative to the tumoral cells, as these parameters at least partially determine the dose deposited on healthy cells crossed by a radiation beam aiming at depositing a target fraction dose (Dmtj) onto a volume containing tumoural cells. US 2012/136194 A1 discloses a treatment planning system for generating a plan for treatment with charged particles beams of a target tissue comprising tumoral cells enclosed within a peripheral surface, which is surrounded by healthy cells, wherein the plan consists of N fractions of irradiation.

It has been observed that the tumoural cells have a longer recovery time to recover from the damages suffered after an irradiation fraction than healthy cells. This allows a treatment plan to span over several fractions distributed over a period of several days and weeks. By ensuring that
(a) the healthy cells receive fraction doses low enough to preserve them,
(b) The tumoural cells receive a total target dose cumulated over the N fractions at least equal to the minimum target dose (DmtT), and
(c) A recovery time between two successive fractions is sufficient to allow substantial recovery of the healthy cells, but not of the tumoural cells,
The treatment plan can ensure that at the end of the treatment, all the tumoural cells have been destroyed, while sparing the healthy cells.

Maximum fraction dose and maximum total dose acceptable to the healthy cells can be defined relative to a Normal Tissue Complication Probability (NTCP) which defines the probability of a given tissue of developing complications upon exposure to a radiation. Values of boundary doses of radiation yielding a given value of the NTCP for a selection of organs are available in the literature. For example, Kehwar, J Cancer Res Ther, Sept.2005, 1(3), 168, lists in Table 3, on p.171, a number of doses yielding a 50% NTCP of developing five years after the treatment symptoms as defined in the last column for a number of organs. The volumes 1/3-3/3 in Table 3 indicate the portion of the total volume of the corresponding organ being irradiated. Alternatively, a medical practitioner can have other sources for determining boundary doses, such as personal experience, or referring to other experimental data.

Historically, treatment plans by radiation therapy included the delivery of radiation doses to the treated cells at a conventional dose deposition rate (CDR) lower than 1 Gy / s. With rare exceptions, current radiation therapy facilities deliver dose-rates around 0.1 Gy / s and most clinical protocols involve daily delivery of N target fraction doses (Dmtj) of 2 to 15 Gy cumulated to reach the total target dose (DmtT) which often exceeds the tolerance limit of normal tissues located in the radiation field, thus damaging them together with the tumoural cells. Recently, it has been observed that a same dose (D) had different effects on healthy cells but not on tumoral cells when deposited at conventional dose deposition rates (CDR) or at ultra-high dose deposition rate (HDR); HDR can be one or more orders of magnitude larger than conventional dose deposition rates (CDR) usually applied. Deposition of a charge at ultra-high dose deposition rates (HDR) is also referred to as FLASH-radiotherapy (= FLASH-RT). It has been demonstrated experimentally on animals and on various organs, that ultra-high rate dose deposition at HDR can significantly spare healthy tissues in comparison with conventional deposition of a same dose at CDR and, at the same time, tumoural cells respond same or even better to HDR deposition than to CDR deposition. For example, FLASH-RT reportedly elicits in mice a dramatic decrease of the incidence of lung fibrosis, of memory loss subsequent to brain irradiation, and of necrosis of the small intestine whilst keeping the anti-tumour efficiency unchanged. Such specific normal tissue sparing has been confirmed in large animals and a patient with cutaneous lymphoma has already been treated with FLASH-RT. Many treatment centres, however, do not dispose of equipment capable of delivering dose at HDR in a time of the order of the ms or s or dispose of equipment that can be modified to deliver HDR only in a very limited field size.

The challenge of generating ever more efficient treatment plans using existing equipment, with enhanced killing of tumoural cells, while sparing as much as possible healthy tissues remains open. The discovery of the differentiated biological reactions of tumoural and healthy cells to radiation doses deposited at ultra-high rates (HDR) provides an additional tool for enhancing the efficacy of the treatment plans. For example, WO2019018813 describes a treatment plan for treating tumours by administering radiations at ultra-high dose deposition rates and a therapeutic agent to a patient with cancer. The treatment plan provides the dual benefits of anti-tumour efficacy plus normal tissue protection when combining therapeutic agents with ultra-high dose deposition rate radiations to treat cancer patients.

The present invention proposes a treatment planning system (TPS) for generating a plan for treatment with charged particles beams, preferably with proton beams, of a target tissue comprising tumoural cells. The plan comprises combining N fractions (N ≥ 1), each fraction including,
- irradiation of volumes including tumoral cells and/or healthy cells at a conventional dose deposition rate (CDR), defined as a dose rate, CDR < 1 Gy / s, and / or
- irradiation of volumes including tumoral cells and/or healthy cells at an ultra-high dose deposition rate (HDR), defined as a dose rate, HDR ≥ 1 Gy / s.

By carefully combining selecting doses deposited at CDR and HDR to tumoural cells and / or to healthy cells, an optimal plan can be achieved with efficient killing of tumoural cells and high sparing of healthy tissues. These and other advantages of the present invention are explained more in detail in the following sections.

### SUMMARY OF THE INVENTION

The objectives of the present invention have been reached with a treatment planning system (TPS) for generating a plan for treatment with charged particles beams, preferably with proton beams, of a target tissue comprising tumoral cells enclosed within a peripheral surface, which is surrounded by healthy cells and/or encloses also healthy cells, wherein the healthy cells form healthy tissues. The plan consists of N fractions of irradiation, with N ≥ 1, and the plan comprises fulfilling the following criteria,
(C1) at the end of the N fractions, all tumoral cells of the target tissue (3t) must have received a total target dose (DmtT) equal to the sum of target fraction doses (Dmtj) received at each fraction (i.e., DmtT = Σ Dmtj), which is at least equal to a minimum target dose (DmtT0) for killing the tumoral cells, i.e., DmtT = Σ Dmtj ≥ DmtT0,
(C2) at the end of the N fractions, all healthy cells (3hi) of the healthy tissues surrounding or enclosed within the peripheral surface must have received a total healthy dose (DhTi) equal to the sum of the healthy fraction doses (Dhij) received at each fraction j (i.e., DhTi = Σ Dhij), such that,
   (C2.1) the healthy fraction dose (Dhij) received at each fraction j does not exceed a predefined threshold for preserving the healthy cells at the end of the fraction j, and
   (C2.2) the total healthy doses (DhTi) received and cumulated at the end of the N fractions does not exceed a predefined threshold for preserving the healthy cells at the end of the treatment of N fractions,

According to the present invention, the plan combines N fractions including,
- irradiation of volumes including tumoral cells and/or healthy cells at a conventional dose deposition rate (CDR), defined as a dose rate, CDR < 1 Gy / s, and / or
- irradiation of volumes including tumoral cells and/or healthy cells at an ultra-high dose deposition rate (HDR), defined as a dose rate, HDR ≥ 1 Gy / s, wherein

The total target dose (DmtT) delivered to and cumulated in a volume of the target tissue at the end of N fractions is defined to fulfill criterion (C1) as a sum over the N fractions j, DmtT = Σ Dmtj = Σ (Dctj + Dhtj) ≥ DmtT0, of target fraction doses (Dmtj) delivered to the target tissues at each fraction, wherein the target fraction dose (Dmtj) delivered during a fraction j is defined as a sum of,
- conventional target doses (Dctj) delivered to that volume at conventional dose deposition rates (CDR) during the fraction j, and of
- high rate target doses (Dhtj) delivered to that volume at ultra-high dose deposition rates (HDR) during the fraction j,

The healthy fraction dose (Dhij) delivered to a volume of healthy cells of a healthy tissue (3i) during a fraction j is equal to a sum, Dhij = Dchij + Dhhij, of
- a conventional healthy fraction dose (Dchij) delivered to that volume at a conventional dose deposition rate (CDR), and of
- a high rate healthy fraction dose (Dhhij) delivered to that volume at ultra-high dose deposition rate (HDR),

The gist of the present invention comprises an equivalent healthy fraction dose (Dmhij) defined as a sum Dmhij = (Dchij + α Dhhij), of
- the conventional healthy fraction dose (Dchij) and
- a product of an equivalence coefficient (α) and of the high rate healthy fraction dose (Dhhij),
does not exceed a maximum equivalent healthy fraction dose (Dmh0ij), for preserving the healthy cells after each fraction j (i.e., Dmhij ≤ Dmh0ij), and thus fulfilling criterion (C2.1), wherein the maximum equivalent healthy fraction dose (Dmh0ij) is smaller than or equal to a maximum healthy conventional fraction dose (Dch0ij) corresponding to a dose deposited onto a healthy cell at a conventional dose deposition rate (CDR) only and yielding a predefined probability of complication (NTCP0i) of the healthy cell (i.e., Dmh0ij ≤ Dch0ij)

The gist of the present invention also comprises a total equivalent healthy dose (DmhTi) delivered to and cumulated in a volume of a healthy tissue at the end of N fractions is defined as a sum over the N fractions j, DmhTi = Σ Dmhij = Σ (Dchij + α Dhhij) with α < 1, preferably α > 0.5, of the equivalent healthy fraction doses (Dmhij), wherein the total equivalent healthy dose (DmhTi) does not exceed a maximum equivalent healthy dose (DmhT0i), for preserving the healthy cells at the end of the N fractions (i.e., DmhTi ≤ DmhT0i ), and thus fulfilling criterion (C2.2), wherein the maximum equivalent healthy dose (DmhT0i) is smaller than a maximum healthy conventional dose (DchT0i) corresponding to a dose deposited onto a healthy cell at a conventional dose deposition rate (CDR) only and yielding a predefined probability of complication (NTCP0i) of the healthy cell (3hi) (i.e., DmhT0i < DchT0i)

The equivalent healthy fraction dose (Dmhij) and the total equivalent healthy dose (DmhTi) are defined as equivalent doses yielding a same or lower probability of complication of a healthy cell (NTCP0i) as if the healthy fraction dose (Dhij) and the total healthy dose (DhTi) had, respectively, been deposited onto the healthy cell at a conventional dose deposition rate (CDR) only.

In an embodiment of the present invention, if a volume of a healthy tissue is irradiated during a fraction j,
- at a conventional dose deposition rate (CDR) only, then Dmhij = Dchij ≤ Dch0ij for that volume at that fraction, or
- at an ultra-high rate (HDR) only, then Dmhij = α Dhhij ≤ Dch0ij and Dhhij ≤ Dhh0ij for that volume at that fraction, wherein Dhh0ij is the maximum high rate healthy fraction dose delivered at HDR only at a single fraction j, not to be exceeded for preserving the healthy cells of the healthy tissue (3i), or
- at both conventional dose deposition rate (CDR) and at ultra-high dose deposition rate (HDR), Dmhij = Dchij + α Dhhij ≤ Dch0ij and Dhij = Dchij + Dhhij < Dhh0ij.

The equivalence coefficient α is not necessarily constant. For example, α can either,
- be a constant, preferably equal to 1 / k, wherein k = 1.2 to 1.5 (i.e., α = 1 / k, with k = 1.2 to 1.5), and preferably, α < 0.9, more preferably α < 0.85, most preferably α < 0.7, or
- depend on a HDR-proportion, xH = Dhhij / (Dchij + Dhhij) of doses deposited at ultra-high rate (HDR), and varying between 1 / k for xH = 1 (i.e., at HDR only), and 1 for xH = 0 (i.e., at CDR only).

The values of the boundary doses Dch0ij and Dhh0ij, as well as DchT0i and DhhT0i not to be exceeded to preserve the healthy cells can be determined by different ways. Dch0ij and DchT0i can predefined by a medical practitioner (clinician) or defined in the literature. Dhh0i and DhhT0i, on the other hand, can be either
- predefined by a medical practitioner or defined in the literature for given values of the HDR, or
- respectively defined as Dhh0ij = k Dch0ij and Dhh0Ti = k DchT0i, for given values of the HDR, wherein k is comprised between 1.2 and 1.5 (i.e., k = 1.2 to 1.5), or
- defined by a pre-established curve of a Normal Tissue Complication Probability (NTCP) for the healthy tissue as a function of dose and dose rate, and wherein Dhh0ij and DhhT0i are defined as the doses delivered at HDR only having same NTCP as the doses Dch0ij and as DchT0i, respectively, delivered at CDR only.

For example, the maximum healthy conventional dose (DchT0i) and the maximum healthy high-rate dose (DhhT0i) at the end of the N fractions can be defined as follows for a selection of healthy tissues

| Healthy tissue (3hi) | DchT0i (Gy) | DhhT0i (Gy) |
|---|---|---|
| brain | 60 | 90 |
| heart | 50 | 75 |
| larynx | 80 | 120 |
| Liver | 40 | 60 |
| Lung | 25 | 38 |
| Stomach | 65 | 98 |
| Eye lens | 18 | 27 |

In one embodiment, all healthy tissues located upstream from the peripheral surface are irradiated at conventional dose deposition rates only during the N fractions, and wherein,
- the healthy fraction dose (Dhij) deposited in said healthy tissues at each fraction j (is equal to the conventional healthy fraction dose (Dchij) and) does not exceed the maximum healthy conventional fraction dose (Dch0ij), and
- the total healthy dose (DhTi) deposited in said healthy tissues at the end of the N fractions (is equal to the conventional healthy dose (Dchi) and) does not exceed the maximum healthy conventional dose (Dch0Ti),
wherein a healthy cell is located upstream of the peripheral surface when a charged particles beam defined in the TPS traverses said healthy cell before penetrating into the peripheral surface.

In an embodiment, all healthy tissues located downstream of the peripheral surface are irradiated at ultra-high dose deposition rates only, and wherein,
- the healthy fraction dose (Dhij) deposited in said healthy tissues at each fraction j (is equal to the high-rate healthy fraction dose (Dhhij) and) does not exceed the maximum high rate healthy fraction dose (Dhh0ij), and
- the total healthy dose (DhTi) deposited in said healthy tissues at the end of the N fractions (is equal to the high-rate healthy dose (DhhTi) and) does not exceed the maximum healthy conventional dose (Dch0Ti),
wherein a healthy cell is located downstream of the peripheral surface when a charged particles beam traverses said healthy cell only after exiting the peripheral surface.

In a preferred embodiment, one or more of the fractions of the plan include the following steps carried out in any sequential order,
- a first step of delivering a conventional dose at a conventional dose deposition rate (CDR) with a first charged particles beam or group of charged particles beams parallel to a first direction each formed by various beamlets, such that,
   o a conventional target fraction dose (Dctj) is delivered at each or some of the one or more fractions to at least 90%; preferably at least 95%, more preferably at least 99% of the tumoral cells of the target tissue (3t), and
   o a conventional healthy fraction dose (Dchi) lower than the maximum healthy conventional fraction dose (Dch0ij) is delivered at each or some of the one or more fractions to the healthy tissues (3hi) surrounding and enclosed within the peripheral surface (i.e., Dchij < Dch0ij), and
- a second step, following the first step, of delivering a high rate dose at an ultra-high dose deposition rate (HDR) with a second charged particles beam or group of charged particles beams parallel to a second direction same as or different from the first direction, such that,
   o a high rate target fraction dose (Dhtj) is delivered at each or some of the one or more fractions to the target tissue such that 100% of the tumoral cells of the target tissue have received a fraction dose at conventional and / or at ultra-high rates (CDR, HDR), and
   o a high rate healthy fraction dose (Dhhij) is delivered at each or some of the one or more fractions to the healthy tissues surrounding and within the peripheral surface, such that the equivalent healthy fraction dose (Dmhij = Dchij + α Dhhij) delivered at both conventional and ultra-high rates (CDR, HDR) to all healthy cells, does not exceed the maximum conventional healthy fraction dose (Dmh0ij) (i.e. Dmhij ≤ Dch0ij).

The second charged particles beam or group of charged particles beams may be selected such as not to overlap with the first charged particles beam or group of charged particles beams in any healthy tissue, surrounding or within the peripheral surface.

In an alternative preferred embodiment, the plan includes a single charged particles beam (PB) or group of beams parallel to a first direction formed by beamlets, such that,
- a conventional target fraction dose (Dctj) is delivered to at least 90%, preferably at least 95%, more preferably at least 99% of the tumoral cells of the target tissue,
- a conventional healthy fraction dose (Dchij) lower than the maximum healthy conventional fraction dose (Dch0ij) is delivered to the healthy tissues (3hi) surrounding and enclosed within the peripheral surface (i.e., Dchij < Dch0ij),
- a high rate target fraction dose (Dhtj) is delivered at each or some of the one or more fractions to the target tissue such that 100% of the tumoral cells of the target tissue have received a fraction dose at conventional and / or at ultra-high rates (CDR, HDR), and
- a high rate healthy fraction dose (Dhhij) is delivered at each or some of the one or more fractions to the healthy tissues surrounding and within the peripheral surface, such that the equivalent healthy fraction dose (Dmhij = Dchij + α Dhhij) delivered at both conventional and ultra-high rates (CDR, HDR) to all healthy cells, does not exceed the maximum conventional healthy fraction dose (Dch0ij) (i.e. Dmhij ≤ Dch0ij).

The peripheral surface may comprise an uncertain volume defining an uncertain zone, defined as a zone comprising both tumoural and healthy cells intermixed at different ratios. In this case, the TPS can comprise the delivery to an entirety of the uncertain volume at the end of N fractions of a total healthy dose (DhTi) such that,
- the total healthy dose (DhTi = DchTi + DhhTi) is larger than the minimum target dose (DmtT0), and lower than the maximum high rate healthy dose (DhhT0i) (i.e., DmtT0 < DhTi < DhhT0i), and
- a total equivalent healthy dose (DmhTi = DchTi + α DhhTi) delivered at the end of the N fractions is lower than the maximum healthy conventional dose (DchT0i) of the healthy tissue (3hi) present in the uncertain zone (i.e., DmhTi < DchT0i), and
- an equivalent healthy fraction dose (Dmhij = Dchij + α Dhhij) delivered at each fraction j is lower than the maximum healthy conventional fraction dose (DchT0ij) of the healthy tissue (3hi) present in the uncertain zone, (i.e., DmhTi < DchT0i).

The TPS of the present invention can a proton beam as define the charged particle beam, which can be delivered in single- or double-scattering mode, or by pencil beam scanning (PBS)

### BRIEF DESCRIPTION OF THE FIGURES

On these figures,
**Fig. 1** (a) shows the energy deposition onto tissues as a function of depth (z) of penetration, for X-rays (XR), and for proton beamlets, adding up to form a sum of individual Bragg Peaks (SOBP) spanning over the whole depth of the tumour (3), 1(b) shows a cut parallel to a proton beam direction crossing a target tissue comprising tumoral cells, and 1(c) shows the corresponding SOBP.

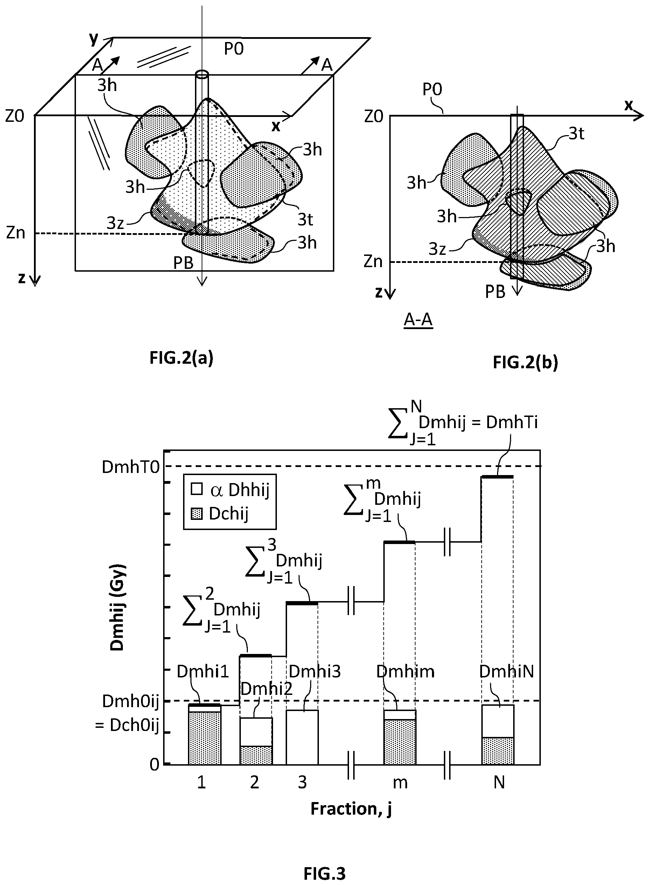

**Fig. 2** (a) illustrates a target tissue (3t) comprising tumoral cells enclosed within a peripheral surface, which is surrounded by healthy cells (3hi) and/or encloses also healthy cells (3hi), 2(b) illustrates a cut along a plane (x, z) parallel to the charged particles beam of the target tissue of Figure 2(a).
**Fig. 3** shows a portion of a treatment plan referring to the healthy tissues (3hi) and divided in N fractions, each fraction comprising delivery of a predefined dose (Dhij) at conventional and/or ultra-high rate (CDR, HDR) cumulating to a total equivalent healthy dose (DmhTi) at the end of the N^{th} fraction.

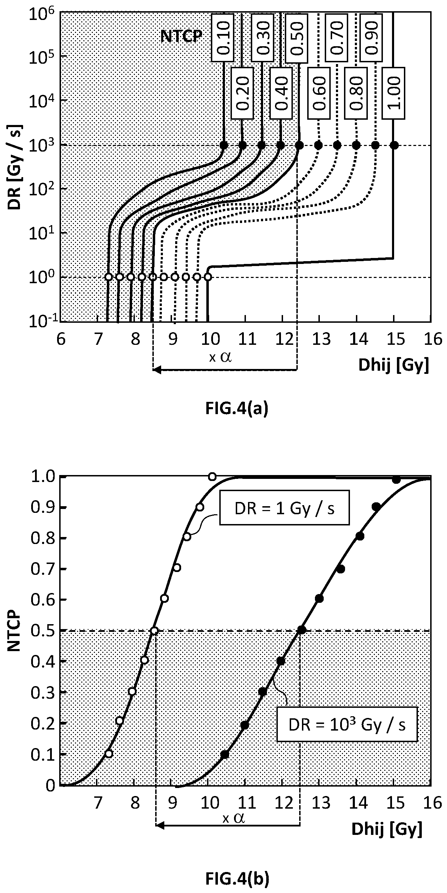

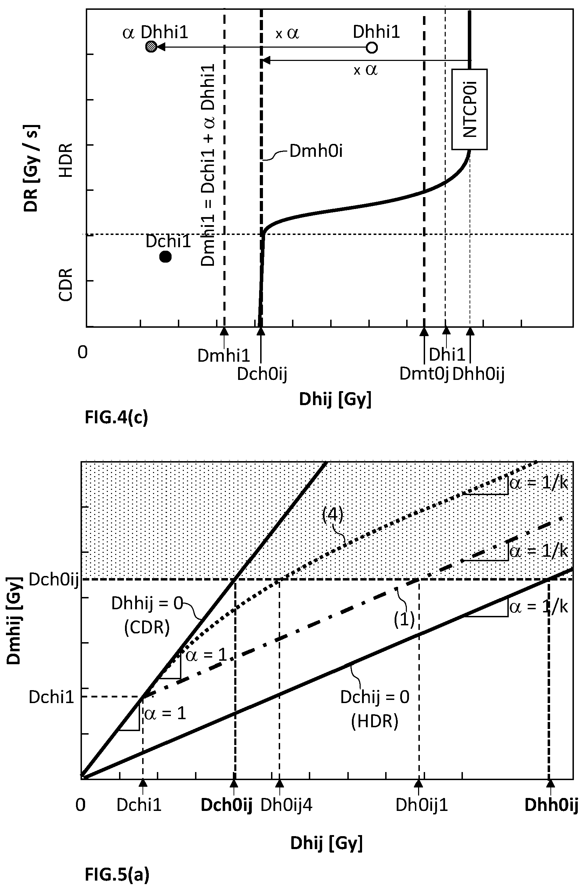

**Fig. 4** (a) shows iso-NTCP-lines as a function of dose (D) deposited into a healthy tissue and of the dose deposition rate (DR), 4(b) NTCP as a function of dose (D) deposited into a healthy tissue at CDR (white circles) and at HDR (black circles), and 4(c) shows the principle of the equivalent healthy fraction dose (Dmhij = Dchij + α Dhhij).

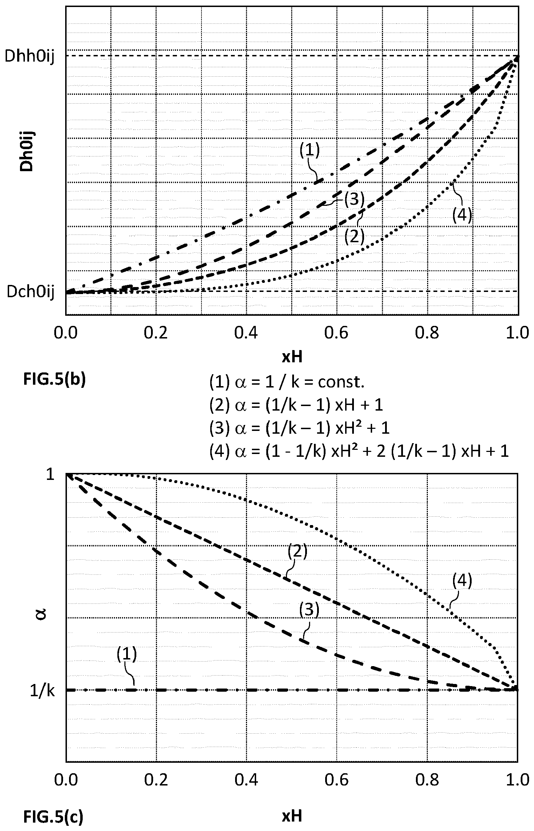

**Fig. 5** illustrates (a) Dmhij (= Dchij + α Dhhij) as a function of Dhij (= Dchij + Dhhij) for a dose deposition only at CDR (= "Dhhij = 0 (CDR)"), only at HDR (= "Dchij = 0 (HDR)"), and a linear (= "(1)") and non-linear (= "(4)") assumptions for a mixed deposition mode with a dose Dch1 ij deposited at CDR, and the rest of the dose deposited at HDR, (b) Dh0ij as a function of xH, for four options of α = f(xH), as represented in Figure 5(c).

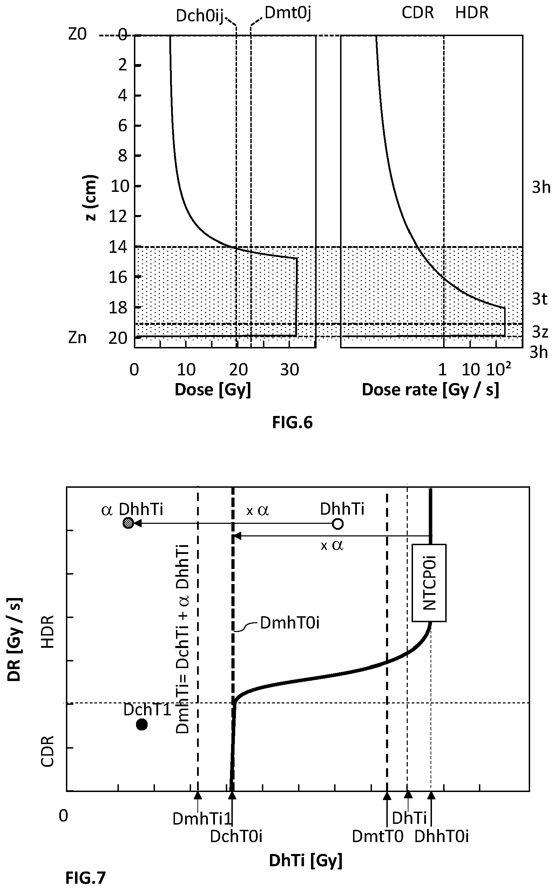

**Fig. 6** shows a treatment plan including a dose (D) deposition profile as a function of depth of penetration (z), as well as a dose deposition rate (DR) profile as function of the same depth of deposition.
**Fig.7** shows the principle of the equivalent healthy dose (DmhTi = DchTi + α DhhTi).

### DETAILED DESCRIPTION.

The present invention concerns a treatment planning system for generating a plan for treatment with charged particles beams of a target tissue (3t). Examples of target tissues are illustrated in Figures 1(b), 2(a), and 2(b), comprising tumoral cells enclosed within a peripheral surface, which is surrounded by healthy cells and/or encloses also healthy cells, wherein the healthy cells form healthy tissues (3hi) wherein the subscript i designates the type of healthy tissues (3hi). Because of the complexity of the multi-parameter treatment to be planned, the TPS generally comprises a computer or processor configured for optimizing the nature and number of beams or beamlets required for fulfilling predefined criteria. The nature of the beam includes the type of charged particles, the intensity of the beam, the doses to be deposited, the dose deposition rates, the direction of the beams, the number and frequency of fractions, and the like. Because of the dose deposition profile including a Bragg peak as illustrated in Figures 1(a) and 1(c), the charged particles are preferably protons. The charged particle beam can be delivered in single- or double-scattering mode, or by pencil beam scanning (PBS). As shown in Figure 3, the plan consists of N fractions of irradiation, with N ≥ 1, the TPS must optimize the nature and number of beam irradiations fulfilling the following criteria,
(C1) at the end of the N fractions, all tumoral cells of the target tissue (3t) must have received a total target dose (DmtT) equal to the sum of target fraction doses (Dmtj) received at each fraction (i.e., DmtT = Σ Dmtj), which is at least equal to a minimum target dose (DmtT0) for killing the tumoral cells, i.e., DmtT = Σ Dmtj ≥ DmtT0,
(C2) at the end of the N fractions, all healthy cells (3hi) of the healthy tissues surrounding or enclosed within the peripheral surface must have received a total healthy dose (DhTi) equal to the sum of the healthy fraction doses (Dhij) received at each fraction j (i.e., DhTi = Σ Dhij), such that,
   (C2.1) the healthy fraction dose (Dhij) received at each fraction j does not exceed a predefined threshold for preserving the healthy cells at the end of the fraction j, and
   (C2.2) the total healthy doses (DhTi) received and cumulated at the end of the N fractions does not exceed a predefined threshold for preserving the healthy cells at the end of the treatment of N fractions

While the FLASH effect is now commonly acknowledged by the persons skilled in the art, the chemical mechanisms underlying the FLASH effect are still elusive. Several different theories have been advanced. For example, it has been proposed that the ultra-high dose deposition rate FLASH-RT significantly reduces the killing of cells circulating in the bloodstream, suggesting that the threshold dose deposition rate separating CDR and HDR could depend on the blood circulation time for one cycle. For humans, a threshold dose deposition rate of about 1 Gy / s has been reported for the FLASH effect to appear. It has also been proposed that organic peroxyl radicals ROO• formed by addition of *O*₂ to primary carbon-centred radicals could play a major role in radio-induced complications. In another example, it has also been reported that the competition between the radio-induced oxygen depletion and the oxygen rediffusion from capillaries could explain the FLASH effect. The present invention is not bound by any one of these or other theories and is based on the experimental evidence that the FLASH effect actually exists. The present invention was developed based on the following observations.
(O1) Tumoral cells are killed similarly, independently of the dose deposition rate (DR).
(O2) A predefined Normal Tissue Complication Probability (NTCP0i) of a healthy tissue (3hi) not to be exceeded is reached at lower doses deposited at conventional dose deposition rates (CDR) than it is at ultra-high dose deposition rates (HDR), as shown in Figures 4(a) to 4(c).
(O3) Tumoral cells in target tissues (3t) have a longer recovery time than healthy tissues exposed to a same radiation dose, regardless of the dose deposition rate.

In order to fulfil the foregoing criteria and as illustrated in Figure 3, the TPS of the present invention combines N fractions including,
- irradiation of volumes including tumoral cells and/or healthy cells at a conventional dose deposition rate (CDR), defined as a dose rate, CDR < 1 Gy / s, and / or
- irradiation of volumes including tumoral cells and/or healthy cells at an ultra-high dose deposition rate (HDR), defined as a dose rate, HDR ≥ 1 Gy / s.

Based on observation (O1) supra, the total target dose (DmtT) delivered to and cumulated in a volume of the target tissue (3t) at the end of N fractions is defined to fulfill criterion (C1) as a sum over the N fractions j of total target fraction doses (Dmtj) delivered to the target tissues (3t) at each fraction. The target fraction dose (Dmtj) delivered during a fraction j is defined as a sum of
- conventional target doses (Dctj) delivered to that volume at conventional dose deposition rates (CDR) during the fraction j, and of
- high rate target doses (Dhtj) delivered to that volume at ultra-high dose deposition rates (HDR) during the fraction j,
This can be expressed as follows: DmtT = Σ Dmtj = Σ (Dctj + Dhtj) ≥ DmtT0, which fulfils criterion (C1).

The healthy fraction dose (Dhij) delivered to a volume of healthy cells of a healthy tissue (3i) during a fraction j is equal to a sum, Dhij = Dchij + Dhhij, of
o a conventional healthy fraction dose (Dchij) delivered to that volume at a conventional dose deposition rate (CDR), and of
o a high rate healthy fraction dose (Dhhij) delivered to that volume at ultra-high dose deposition rate (HDR),

Based on observation (O2) supra, that a given NTCP0i not to be exceeded of a healthy cell is reached at lower conventional healthy doses (Dchij) deposited at CDR than it is at HDR, an equivalent healthy fraction dose (Dmhij) is defined as a sum Dmhij = Dchij + α Dhhij, of
o the conventional healthy fraction dose (Dchij) and
o a product of an equivalence coefficient (α) and of the high rate healthy fraction dose (Dhhij),

The equivalent healthy fraction dose (Dmhij) is defined as an equivalent dose yielding a same or lower probability of complication of a healthy cell (3hi) (NTCP0) as if the healthy fraction dose (Dhij) had been deposited onto the healthy cell at a conventional dose deposition rate (CDR) only. This definition is a direct consequence of observation (O2) supra.

In order to fulfil the criterion (C2.1) supra, the equivalent healthy fraction dose (Dmhij) must not exceed a maximum equivalent healthy fraction dose (Dmh0ij), for preserving the healthy cells after each fraction j (i.e., Dmhij ≤ Dmh0ij). The maximum equivalent healthy fraction dose (Dmh0ij) is smaller than or equal to a maximum conventional healthy fraction dose (Dch0ij) corresponding to a dose deposited onto a healthy cell at a conventional dose deposition rate (CDR) only and yielding a predefined probability of complication (NTCP0i) of the healthy cell (3hi) (i.e., Dmh0ij ≤ Dch0ij). Dmh0ij can be smaller than Dch0ij to allow for a safety margin in case the equivalence coefficient α for a specific healthy tissue (3hi) is not defined with sufficient certainty or accuracy.

In order to fulfil the criterion (C2.2) supra, a total equivalent healthy dose (DmhTi) delivered to and cumulated in a volume of a healthy tissue (3hi) at the end of N fractions is defined as a sum over the N fractions j, DmhTi = Σ Dmhij = Σ (Dchij + α Dhhij) with α ≤ 1, preferably α > 0.5, of the equivalent healthy fraction doses (Dmhij). The total equivalent healthy dose (DmhTi) must not exceed a maximum equivalent healthy dose (DmhT0i), for preserving the healthy cells at the end of the N fractions (i.e., DmhTi ≤ DmhT0i).

The total equivalent healthy dose (DmhTi) is defined as an equivalent dose yielding a same or lower probability of complication of a healthy cell (3hi) (NTCP0) as if the total healthy dose (DhTi) had been deposited onto the healthy cell at a conventional dose deposition rate (CDR) only. This definition is a direct consequence of observation (O2) supra. The maximum equivalent healthy dose (DmhT0i) is smaller than or equal to a maximum conventional healthy dose (DchT0i) corresponding to a dose deposited onto a healthy cell at a conventional dose deposition rate (CDR) only and yielding a predefined probability of complication (NTCP0i) of the healthy cell (3hi) (i.e., DmhT0i ≤ DchT0i). Here again, DmhT0i can be smaller than DchT0i to allow for a safety margin in case the equivalence coefficient α for a specific healthy tissue (3hi) is not defined with sufficient certainty or accuracy.

### OBSERVATION (O2): HEALTHY DOSE Dhij(NTCP0i) IS A FUNCTION OF DOSE DEPOSITION RATE (DR)

Figures 4(a) to 4(c) illustrate graphically the dependence on the dose deposition rate (DR) of the healthy dose (Dhij) required for reaching a given NTCP for a given healthy tissue (3hi) during a fraction j of the treatment. Note that the expression *"healthy dose"* is used herein as a contraction of *"dose delivered to a healthy cell,"* and is not to be understood as a dose which is beneficial to the health of a cell. According to the criteria (C2.1) and (C2.2) the *"healthy doses"* must be such as to limit as much as possible the damages to the healthy cells traversed by beams of charged particles whilst, at the same time the tumoral cells of the target tissue (3t) are killed by the same beams.

Figure 4(a) shows iso-NTCP lines in a plot of the dose deposition rate (DR) as a function of healthy dose (Dhij) for values of NTPC comprised between 10% and 100% probability. Assuming the value NTCP0i of the NTCP not to be exceeded during a fraction j of the treatment is 50% probability of complication, then doses left of the iso-NTCP line labelled (0.5) represented by a shaded area can be deposited at corresponding dose deposition rates. It can be seen that the dose (D) defining the iso-NTCP line = 50% strongly depends on the rate at which the dose is being deposited.

Figure 4(b) is a direct consequence of Figure 4(a), wherein the white dots aligned at the ordinate DR = 1 Gy / s of Figure 4(a) are reported in Figure 4(b) showing the doses (Dhij) required for reaching a given NTCP at said dose rate, yielding a sigmoid curve of white dots. The same exercise was repeated with the black dots aligned at the ordinate DR = 10³ Gy / s of Figure 4(a) which are reported in Figure 4(b) yielding the sigmoid curve of black dots. Taking NTCP0i = 50% as in Figure 4(a), yields a corresponding shaded area indicative of the doses which can be deposited onto healthy cells of a healthy tissue (3hi) at these two deposition rates.

The iso-NTCP lines in Figures 4(a) and 4(b) define the doses (Dhij) required to reach a given NTCP when the whole of each dose is delivered at the corresponding dose deposition rate (DR). In other words, in an embodiment of the invention, if a volume of a healthy tissue (3hi) is irradiated during a fraction j,
- at a conventional dose deposition rate (CDR) only, then the equivalent healthy fraction dose (Dmhij) is equal to the conventional healthy fraction dose (Dchij) (i.e., Dmhij = Dchij), which must be smaller than or equal to the maximum conventional healthy fraction dose (Dch0ij) (i.e., Dmhij = Dchij ≤ Dch0ij) for that volume at that fraction j, or
- at an ultra-high rate (HDR) only, then
   ∘ the equivalent healthy fraction dose (Dmhij) is equal to the product of the equivalence coefficient (α) and of the high rate healthy fraction dose (Dhhij) (i.e., Dmhij = α Dhhij), which must be smaller than or equal to the maximum conventional healthy fraction dose (Dch0ij) (i.e., Dmhij = α Dhhij ≤ Dch0ij) and
   ∘ the high rate healthy fraction dose (Dhhij) must be smaller than or equal to a maximum high rate healthy fraction dose (Dhh0ij) (i.e., Dhhij ≤ Dhh0ij) for that volume at that fraction, wherein Dhh0ij is the maximum high rate healthy fraction dose delivered at HDR only at a single fraction j, not to be exceeded for preserving the healthy cells of the healthy tissue (3i).

The present invention may, however, comprise fractions j combining doses deposited at both conventional dose deposition rates (CDR) and ultra-high dose deposition rates (HDR). At both conventional dose deposition rate (CDR) and at ultra-high dose deposition rate (HDR), the equivalent healthy fraction dose (Dmhij) is equal to the sum of the conventional healthy fraction dose (Dchij) and of the product of the equivalence coefficient α and of the high rate healthy fraction dose (Dhhij) (i.e., Dmhij = Dchij + α Dhhij), which must be smaller than or equal to the maximum conventional healthy fraction dose (Dch0ij) (i.e., Dmhij = Dchij + α Dhhij ≤ Dch0ij).

This is illustrated in Figure 4(c), wherein the black dot labelled "Dchi1" is a dose deposited onto healthy cells at CDR and the white dot labelled `Dhhi1" is a dose deposited at HDR onto the same healthy cells during a same fraction j = 1. Note that in the example of Figure 4(c), Dchi1 < Dmhi1 < Dch0ij < Dhhi1 < Dhi1 < Dhh0ij. Strictly speaking, the present invention is satisfied as long as Dmhij < Dch0ij, and Dhij is necessarily smaller than Dhh0ij. Note that Dhhij is necessarily smaller than Dhij, or it can be equal to Dhij if and only if Dchij = 0 (i.e., deposition at HDR only). The healthy fraction dose (Dhij) can be greater or smaller than or equal to Dch0i, as long as the equivalent healthy fraction dose (Dmhij) is smaller than or equal to Dch0i. Contrary to what could be intuitively expected, the healthy fraction dose Dhij = Dchij + Dhhij can be substantially larger than the maximum conventional healthy fraction dose (Dch0ij) as is the case in the example of Figure 4(c).

The maximum conventional healthy fraction dose (Dch0ij) is generally the predefined threshold value given by a clinician as criterion for establishing a treatment plan which preserves the healthy cells at the end of the fraction j (= criterion (C2.1)). Though greater than Dch0ij, the healthy fraction dose (Dhi1) surprisingly fulfils the criterion (C2.1). This is made possible because the equivalent healthy fraction dose ("α Dhhi1") of the dose (Dhhi1) deposited at ultra-high rate only is only a fraction defined by the equivalence coefficient α of the high rate healthy fraction dose (Dhhi1) actually deposited at HDR. The corresponding equivalent healthy fraction dose is indicated Figure 4(c) with a shaded dot labelled "α Dhhi1" and is obtained by multiplying the dose Dhhi1 by the equivalence coefficient α. The equivalent healthy fraction dose (Dmhi1) is indicated by a dashed line and is simply the sum, Dmhi1 = Dchi1 + α Dhhi1. It can be seen that in the example of Figure 4(c), the equivalent healthy fraction dose (Dmhi1) is smaller than the maximum conventional healthy fraction dose (Dch0ij) thus fulfilling the criterion (C2.1) for the fraction j = 1 illustrated therein. To preserve the healthy tissues (3i), care must be taken that the total healthy dose (Dhi1) does not exceed a maximum healthy fraction dose (Dh0ij) discussed in continuation.

In many cases as illustrated in Figure 4(c), a minimum target fraction dose (Dmt0j) required to kill tumoral cells in a volume irradiated during a fraction j can be comprised between Dch0ij and Dhh0ij. A great advantage of the present invention is that a volume comprising both tumoral cells and healthy cells can be radiation treated during a fraction j = 1 by deposition of a given fraction dose (Dhi1 = Dmt1) at combined deposition rates of CDR and HDR, killing the tumoral cells, because the target fraction dose (Dmt1) is greater than the minimum target fraction dose (Dmt0j) and, at the same time, preserving the healthy cells comprised in the irradiated volume, because the healthy fraction dose (Dhi1) is such that the total equivalent healthy fraction dose (Dmhi1) is smaller than the maximum healthy conventional fraction dose (Dch0ij). In summary, a volume comprising both tumoral cells and healthy cells can be treated by deposition of a fraction dose (Dhij = Dmtj) at both CDR and HDR provided, that
- Dchij + Dhhij = Dhij = Dmtj > Dmt0j, to kill the tumoral cells in the volume, and
- Dchij + α Dhhij = Dmhij ≤ Dch0ij, to preserve the healthy cells in the volume.

It is clear to a person skilled in the art, that the foregoing discussion of Figure 4(c) for a fraction j = 1 applies *mutatis mutandis* to any fraction j (i.e., the subscript "1" can be replaced by "j").

### EQUIVALENCE COEFFICIENT α

The value of the equivalence coefficient α can be determined for dose depositions at ultra-high dose deposition rates (HDR) by establishing NTCP curves as a function of dose (Dhij) and dose deposition rate (DR) as illustrated for example in Figures 4(a) to 4(c). At sufficiently high dose deposition rates, wherein the dose (Dhh0ij) yielding a predefined NTCP0i becomes independent of the dose rate the factor α becomes substantially constant (i.e., the right-hand vertical portion of the solid curve in Figures 4(a), 4(c), and 7), and can be defined as being equal to 1 / k, wherein k = 1.2 to 1.5 (i.e., α = 1 / k, with k = 1.2 to 1.5), and preferably, α < 0.9, more preferably α < 0.85, most preferably α < 0.7.

Figure 5(a) illustrates various examples of the relationship between the equivalent healthy fraction dose (Dmhij) and the healthy fraction dose (Dhij). The solid lines show the relationship for deposition at CDR only, labelled "Dhhij= 0 (CDR)", and at HDR only, labelled "Dchij = 0 (HDR)". Considering that Dmhij = Dchij + α Dhhij, and that Dhij = Dchij + Dhhij, it follows that,
- at CDR only, Dhhij = 0 and therefore Dmhij = Dhij, definining the straight line of slope 1 labelled "Dhhij =0 (CDR)" of Figure 5(a), which intercepts the horizontal dashed line Dmhij = Dch0ij not to be exceeded to preserve the healthy tissues (3hi) at a value of Dhij = Dch0ij, and
- at HDR only, Dchij = 0 and therefore Dmhij = α Dhhij, defining the straight line of slope α = 1/k labelled "Dchij = 0 (HDR)" of Figure 5(a), which intercepts the horizontal dashed line Dmhij = Dch0ij not to be exceeded to preserve the healthy tissues (3hi) at a value of Dhij = Dhh0ij.

In a treatment plan comprising dose deposition at both conventional and ultra-high rates (CDR, HDR), the relationship between Dmhij and Dhij is comprised between the two straight lines of slope 1 (= "Dhhij =0 (CDR)") and of slope α = 1 / k (= "Dchij = 0 (HDR)") of Figure 5(a). Fixing the conventional healthy dose (Dchij) deposited in a volume during a treatment fraction to Dch1i (i.e., Dchij = Dch1i = const.), then Dchij = Dch1i + α Dhhij, wherein Dhhij is free to vary from 0 (= CDR only) to any positive value. In case the whole dose is deposited at CDR only (i.e., Dhhij = 0), then Dchij = Dhij = Dch1i, which marks the starting point of the curve relating Dmhij and Dhij.

Defining a HDR proportion, xH = Dhhij / (Dchij + Dhhij), ranging from xH = 0, defining deposition at CDR only, to xH = 1, defining deposition at HDR only and defining a proportion of the healthy fraction dose (Dhij) which is deposited at HDR, Dmhij = Dch1i + α Dhhij can also be expressed as a function of Dhij as, Dmhij = (1 + xH (α - 1)) Dhij. The maximum healthy dose (Dh0ij) which can be deposited onto healthy cells of a healthy tissue (3hi) as a function of xH can be determined by equating Dmhij = Dch0ij = (1 + xH (α - 1)) Dh0ij, which is represented graphically in Figure 5(b), for different values of α, depending on whether or not and how it varies as a function of xH, as illustrated in Figure 5(c).

In one embodiment of the invention labelled (1) in Figure 5(c), the equivalence coefficient α is independent of the HDR proportion, xH = Dhhij / (Dchij + Dhhij). The HDR proportion (xH) can also be expressed as, xH = 1 - Dch1i / Dhij. It follows that if the equivalence coefficient α is independent of xH, it is also independent of Dhij. The relation Dchij = Dch1i + α Dhhij therefore defines a straight line of slope α = 1 / k starting at the point Dmhij = Dhij = Dch1i. This embodiment is illustrated in Figures 5(a) and 5(b) with the corresponding dashed lines labelled "(1)" showing linear relationships between Dmhij and Dhij starting at the point Dmhij = Dhij = Dchi1 (cf. Figure 5(a)) and between a non-linear relationship between Dch0ij and xH (cf. Figure 5(b)).

In an alternative embodiment, the equivalence coefficient α depends on the HDR-proportion, xH = Dhhij / (Dchij + Dhhij) of doses deposited at ultra-high rate (HDR), and varies between α = 1 / k for xH = 1 (i.e., at HDR only), and α = 1 for xH = 0 (i.e., at CDR only). Three such embodiments are illustrated in Figure 5(c) as examples, with,
- the short-dashed line (2) defining a linear relationship between α and xH, α = (1/k - 1) xH + 1; this embodiment is illustrated in Figure 5(b) with the corresponding short-dashed line (2);
- the long-dashed line (3) defining a polynomial relationship between α and xH, α = (1/k - 1) xH²+ 1; this embodiment is illustrated in Figure 5(b) with the corresponding long-dashed line (3);
- the dotted line (4) defining a second polynomial relationship between α and xH, α = (1 - 1/k) xH²+ 2 (1/k - 1) xH + 1; this embodiment is illustrated in Figures 5(a) and 5(b) with the corresponding dotted lines labelled "(4)"; Figure 5(a) shows a non-linear relationship between Dmhij and Dhij with a conventional healthy fraction dose Dchij = Dch1ij = constant, starting at Dhij = Dmhij = Dch1ij and intercepting at a value of Dhij = Dhi0ij(4) the predefined threshold (Dch0ij) represented by the horizontal dashed line at Dmhij = Dch0ij not to be exceeded to preserve the healthy tissues (3hi).

Whether the equivalence coefficient α depends or not on the HDR-proportion xH is mostly dependent on the type of healthy tissue (3hi) concerned and a skilled person knows where to find or how to assess this information. The abscissa (Dhij) of Figure 5(a) shows how doses (Dhij) substantially higher than the predefined threshold defined by a clinician with the maximum conventional healthy fraction dose (Dch0ij) can be irradiated without exceeding the predefined NTCP0i value specified for preserving the integrity of a given healthy tissue (3hi), by using HDR, either alone or in combination with CDR. Figure 5(b) shows how the maximum healthy dose (Dh0ij) increases above Dch0ij with increasing values of xH (i.e., with increasing contribution of Dhhij in the radiation treatment). This is of great interest for the following reasons. Although a treatment plan aims at reducing the doses deposited onto healthy cells relative to the doses deposited onto tumoural cells, in practice, this is not always feasible. For example, if an irradiated volume comprises both tumoural and healthy cells, then a substantially same dose is deposited onto both tumoural and healthy cells, such that the target fraction dose (Dmti) is substantially equal to the healthy fraction dose (Dhij) (i.e., Dmtj ≃ Dhij). In most cases, the minimum target fraction dose (Dmt0j) required for killing tumoral cells in a volume in a fraction j is higher than the maximum conventional healthy fraction dose (Dch0ij) (i.e., Dmt0j > Dch0ij). These two competing conditions (viz., Dmtj ≃ Dhij and Dmt0j > Dch0ij) make it very difficult to kill tumoural cells whilst sparing the healthy cells comprised within the volume within the predefined value of the NTCP0i (i.e., if Dmt0j > Dch0ij → problem). Since the tumoural cells kill rate of a dose is substantially independent of the dose deposition rate (cf. 0bservation (O1) supra), and since the NTCP of healthy cells at the same dose decreases with higher deposition rates (cf. Observation (O2) supra), it is possible to deposit a target fraction dose (Dmti) higher than the maximum conventional healthy fraction dose (Dch0ij), yet still keeping within the predefined value of NTCP0i, required for sparing the healthy tissues (3hi).

The window defining the doses which can be deposited in a volume in a fraction j yielding a value of the NTCP ≤ NTCP0i spans between Dch0ij and Dhh0ij. If the whole dose (Dmti) is deposited at CDR only, the target fraction dose (Dmtj) cannot exceed the maximum conventional healthy fraction dose (Dch0ij) which defines the lowest boundary of the window. This situation is representative of the state of the art, with the problem discussed supra in case Dmt0j > Dch0ij. By combining depositions at both CDR and HDR, the maximum healthy dose (Dh0ij) increases with increasing HDR-proportion xH as illustrated in Figure 5(b), depending on the dependency of α vs xH.

The highest allowable dose (Dhij) which can "safely" be deposited onto the healthy tissue in a fraction j is the maximum high rate healthy fraction dose (Dhhij), which can be deposited if the whole target fraction dose (Dmtj) is deposited at HDR (i.e., at xH = 1). If the prescribed target fraction dose (Dmtj) required for killing tumoral cells in a volume in a fraction j increases, it becomes more difficult to deposit the whole dose (Dmti) at HDR only. This is mainly due to limitations of the equipment which may be unable to deposit more than a given dose in a given time of the order of the ms to s. By combining CDR and HDR to deposit the whole target fraction dose (Dmtj) in one fraction j, a treatment plan implementable in existing equipment can be generated wherein the total dose (Dmtj ≃ Dhij) deposited into the volume can be greater than or equal to the minimum target fraction dose (Dmt0j) required for killing tumoral cells in the volume in the fraction j.

If the equivalence coefficient α = 1 / k ≠ f(xH) and is constant, illustrated in Figures 5(a) to 5(c) with the mixed straight line labelled "(1)", then a dose up to a maximum value (Dhi0ij(1)) can be deposited onto healthy cells within the predefined NTCP0i. If the equivalence coefficient α = f(xH) with α = 1 for Dhhij = 0 and α = 1/k for Dhhij = 1, illustrated in Figure 5(a) with the dotted curved line labelled '(4)", a dose up to a maximum value (Dhi0ij(4)) can be deposited onto healthy cells within the predefined NTCP0i. The maximum dose values which can be deposited into a volume comprising healthy cells is therefore defined as follows: Dch0ij < Dhi0ij(4) < Dhi0ij(1) < Dhh0ij.

Provided the maximum dose value which can be deposited in a volume comprising both tumoural and healthy cells is higher than the minimum target fraction dose (Dmt0j), then the irradiation fraction can successfully be carried out, killing the tumoural cells while sparing the healthy cells comprised within the irradiated volume within the predefined NTCP0i.

### MAXIMUM AND MINIMUM DOSES

The treatment plan must fulfil a number of criteria, including,
(C1) DmtT = Σ Dmtj ≥ DmtT0, wherein DmtT is the total target dose required to kill tumoural cells in a volume during a fraction, and DmtT0 is the minimum target dose cumulated over the N fractions to kill all tumoural cells comprised within the peripheral surface,
(C2.1) Dmhij = Dchij + α Dhhij ≤ Dch0ij, wherein Dmhij is the equivalent healthy fraction dose and Dch0ij is the maximum conventional healthy fraction dose not to be exceeded during a fraction to preserve the healthy cells (cf. Figure 4(c)), and
(C2.2) DmhTi = Σ Dmhij = Σ (Dchij + α Dhhij) ≤ DchT0i, wherein DmhTi is the equivalent healthy dose and DchT0i is the maximum conventional healthy dose cumulated over the N fractions j not to be exceeded to preserve the healthy cell (cf. Figure 7).

The criteria (2.1) and (2.2) can also be expressed as,
(C2.1) Dhij = Dchij + Dhhij ≤ Dh0ij, wherein Dhij is the healthy fraction dose and Dh0ij is the maximum healthy fraction dose not to be exceeded during a fraction to preserve the healthy cells (cf. Figure 5(a)), and
(C2.2) DhTi = Σ Dhij = Σ (Dchij + Dhhij) ≤ DhT0i, wherein DhTi is the healthy dose cumulated over the N fractions j, and DhT0i is the maximum healthy dose cumulated over the N fractions j not to be exceeded to preserve the healthy cells.

The minimum target dose (DmtT0) must be exceeded to ensure that the treatment kills the tumoral cells comprised within the peripheral surface at the end of the N fractions j. If the tumoural cells belong to a same tissue or organ as healthy cells (3hi) located within or adjacent the peripheral surface, then the minimum target dose (DmtT0) would most probably be higher than the maximum conventional healthy dose (DchT0i) not to be exceeded in order to spare said healthy cells of same nature as the tumoural cells. This of course constitutes an apparently inescapable trap as it would therefore seem impossible to determine a dose sufficiently high to kill the tumoural cells and sufficiently low to spare the healthy cells adjacent to the tumoural cells. The minimum target dose (DmtT0) is generally predefined by a medical practitioner, defining Criterion (C1).

As can be seen in Figure 5(a), the maximum healthy fraction dose Dh0ij is comprised between the maximum conventional fraction dose (Dch0ij) and the maximum high rate fraction dose (Dhh0ij), i.e., Dch0ij ≤ Dh0ij ≤ Dhh0ij, depending on the HDR proportion, xH = Dhhij / (Dchij + Dhhij). In one embodiment, the maximum healthy fraction dose Dh0ij can be expressed as follows, Dh0ij = Dch0ij + (1 - α) Dhhij(xH), wherein α may or may not vary with xH, and Dhhij(xH) is the high rate healthy fraction dose at a value of xH such that Dmhij = Dch0ij.

Similarly, the maximum healthy dose DhT0i is comprised between the maximum conventional healthy dose (DchT0i) and the maximum high rate healthy dose (DhhT0i), i.e., DchT0i ≤ DhT0i ≤ DhhT0i, depending on the HDR proportion, xH. In one embodiment, DhT0i can be expressed as, DhT0i = DchT0i + (1 -α) DhhTi(xH), wherein α may or may not vary with xH, and DhhTi(xH) is the high rate healthy dose at a value of xH such that DmhTi = DchT0i.

The maximum conventional healthy fraction dose (Dch0ij) and the maximum conventional healthy dose (DchT0i) which define the fraction dose and total cumulated dose deposited at CDR only, yielding a predefined NTCP0i not to be exceeded to preserve the healthy cells and which are used to define Criteria C2.1 and C2.2, can be predefined by a medical practitioner or defined in the literature. For example, Table 1 lists values of DchT0i for a number of organs/ tissues, for a value of NTCP0i = 50% and diagnosed 5 years after the radiotherapy, extracted from Table 3 in Kehwar, *J Cancer Res Ther.,* (Sept. 2005), 1 (3), p.170 . These values are representative of what a medical practitioner could use as reference to define a value of DchT0i.

**Table 1: Examples of DchT0i values for some tissues irradiated at CDR only (Kehwar (2005))**

| **Healthy tissue (3hi)** | **DchT0i (Gy)** | **End point** |
|---|---|---|
| brain | 60 | Necrosis / infraction |
| heart | 50 | Pericarditis |
| larynx | 80 | Cartilage necrosis |
| Liver | 40 | Liver failure |
| Lung | 25 | Pneumonitis |
| Stomach | 65 | Ulceration / perforation |
| Eye lens | 18 | Cataract requiring intervention |
| Ear (Mid / Ext) | 65 | Chronic serious otitis |
| Oesophagus perforation | 68 | Clinical stricture |
| Bladder volume | 79 | Symptomatic bladder contraction |
| Colon | 55 | Obstruction / perforation / ulceration / fistula |
| Spinal cord (20 cm) | 69 | Myelitis / necrosis |
| Femoral head and neck | 65 | Necrosis |
| Optic nerve / optic chiasma | 65 | Blindness |
| Retina | 65 | Blindness |
| Rib cage | 65 | Pathologic fracture |
| Parotid | 46 | Xerostomia |
| Thyroid | 80 | Clinical thyroid is |

The maximum high rate healthy fraction dose (Dhh0ij) and the maximum high rate healthy dose (DhhT0i) which define the fraction dose and total cumulated dose deposited at HDR only, yielding the predefined NTCP0i not to be exceeded to preserve the healthy cells and which can be used in the present invention, can either be,
- predefined by a medical practitioner or defined in the literature for given values of the HDR, or
- respectively defined as Dhh0ij = k Dch0i and Dhh0Ti = k DchT0i, for given values of the HDR, wherein k is comprised between 1.2 and 1.5 (i.e., k = 1.2 to 1.5), or
- defined by a pre-established curve of a Normal Tissue Complication Probability (NTCP) for the healthy tissue (3hi) as a function of dose and dose deposition rate as illustrated in Figures 4(a) to 4(c), and 7, and wherein Dhh0i is defined as the dose having same NTCP as Dch0i.

For example, values of DhhT0i can be estimated from the values of DchT0i listed in Table 1 by multiplying them by a factor k = 1.5. The corresponding results are listed in Table 2.

**Table 2: Examples of DhhT0i values for some tissues irradiated at HDR only DhhT0i = k DchT0i**

| **Healthy tissue (3hi)** | **DchT0i (Gy)** | **End point** |
|---|---|---|
| brain | 90 | Necrosis / infraction |
| heart | 75 | Pericarditis |
| larynx | 120 | Cartilage necrosis |
| Liver | 60 | Liver failure |
| Lung | 38 | Pneumonitis |
| Stomach | 98 | Ulceration / perforation |
| Eye lens | 27 | Cataract requiring intervention |
| Ear (Mid / Ext) | 98 | Chronic serious otitis |
| Oesophagus perforation | 102 | Clinical stricture |
| Bladder volume | 119 | Symptomatic bladder contraction |
| Colon | 83 | Obstruction / perforation / ulceration / fistula |
| Spinal cord (20 cm) | 104 | Myelitis / necrosis |
| Femoral head and neck | 98 | Necrosis |
| Optic nerve / optic chiasma | 98 | Blindness |
| Retina | 98 | Blindness |
| Rib cage | 98 | Pathologic fracture |
| Parotid | 69 | Xerostomia |
| Thyroid | 120 | Clinical thyroidis |

### LOCAL DISTRIBUTION OF DOSE DEPOSITIONS AT CDR AND HDR

For a given irradiation equipment with limited capacity to deliver a given dose at a given ultra-high rate (HDR), the properties of the FLASH effect can be taken advantage of by selecting a volume of the target tissue (3t) to be treated in a fraction j and, depending on the presence and location of healthy cells relative to the volume, to optimize the HDR-proportion xH = Dhhij / (Dchij + Dhhij) of doses deposited at CDR and at HDR, respectively. In one embodiment wherein the charged particles are protons, all healthy tissues (3hi) located upstream from the peripheral surface are irradiated at conventional dose deposition rates only during the N fractions. This is made possible because of the dose deposition profile of proton beams, comprising a low deposition increasing locally to form a Bragg peak. This is represented graphically in Figure 1(b) and 1(c), wherein the healthy tissue (3hi) first traversed by the proton beam represented by a cylinder to take account of a width of the proton beam, receives a low dose, which can "safely" be deposited at CDR within the predefined value of NTCP0i. Of course, the following criteria must still be fulfilled,
- the healthy fraction dose (Dhij) deposited in said healthy tissues at each fraction j does not exceed the maximum conventional healthy fraction dose (Dch0ij), and
- the total healthy dose (DhTi) deposited in said healthy tissues at the end of the N fractions does not exceed the maximum conventional healthy dose (Dch0Ti).

A healthy cell is located upstream of the peripheral surface when a charged particles beam defined in the TPS traverses said healthy cell before penetrating the peripheral surface. Similarly, a healthy cell is located downstream of the peripheral surface when a charged particle beam defined in the TPS traverses said healthy cell only after exiting the peripheral surface. A dose is "safely" deposited onto a healthy cell if the corresponding NTCP ≤ NTCP0i.

On the other hand, all healthy tissues (3hi) located downstream of the peripheral surface can be irradiated at least partially, preferably solely at ultra-high dose deposition rates, wherein,
- the healthy fraction dose (Dhij) deposited in said healthy tissues at each fraction j does not exceed the maximum high rate healthy fraction dose (Dh0ij), and
- the total healthy dose (DhTi) deposited in said healthy tissues at the end of the N fractions does not exceed the maximum conventional healthy dose (Dh0Ti).

Figure 6 illustrates an example of distribution in depth of penetration (z) of a proton beam of the dose (D) deposited in a fraction j, and of the dose deposition rate (DR). The dose (D) defines a portion of low doses deposited onto healthy cells of healthy tissues (3hi) located upstream of the tumoural cells of the target tissue (3t), followed by a SOBP of the type illustrated in Figures 1(a) and 1(c), spanning the whole depth of the target tissue (3t), falling abruptly downstream of the target tissue (3t). The cells located adjacent to the downstream end of the peripheral surface along the proton beam direction are the trickiest, because they potentially receive a high dose (D) of irradiation at the tail of the Bragg peak, and may comprise healthy cells mixed with tumoural cells in an uncertain zone (3z) located within the peripheral surface, or be formed of healthy tissues (3hi) outside of the peripheral surface. For this reason, it is preferred if the dose (D) deposited along the beam passage be deposited at varying deposition rates (DR), with deposition at CDR upstream of and in the upstream portion of the peripheral surface, increasing until reaching HDR as the beam penetrates deeper closer to and crosses the downstream end of the peripheral surface. The deposition rate must be at least partly at HDR when the dose (D) deposited in a volume exceeds the maximum conventional healthy fraction dose (Dch0ij) in case healthy cells are present in the volume. In the example of Figure 6, healthy tissues can be exposed to doses higher than Dch0ij in particular in the uncertain zone (3z) located within a downstream portion of the peripheral surface, and also directly downstream of the peripheral surface. It is important that such doses be deposited at least partly at HDR to satisfy criteria (C2.1) and (C2.2).

### UNCERTAIN ZONE (3z)

The peripheral surface enclosing the tumoral cells of the target tissue (3t) may not form a clear interface separating tumoural cells inside thereof from healthy cells outside thereof, but may form instead an interphase defining an uncertain zone (3z) comprising both tumoural and healthy cells intermixed at different ratios. Figures 2(a), 2(b), and 6 show uncertain zones (3z) located in a downstream portion of the peripheral surface because, as discussed supra, they are the trickiest when irradiating with proton beams. It is clear, however, that such uncertain zones (3z) can be located anywhere along the peripheral surface, or even within the bulk of the target tissue (3t) in case there is an island of healthy cells enclosed within the peripheral surface, as shown in Figures 2(a) and 2(b). To reduce the undesirable side effects of an irradiation treatment, all healthy cells, even those included in an uncertain zone should preferably be spared. These healthy cells are, however, particularly difficult to spare because they are exposed to a same irradiation dose as the tumoural cells, which must be at least equal to the minimum target fraction dose (Dmt0j). For this reason, to date most clinician may favour certainty of destroying the tumoural cells, to the expenses of any healthy cells comprised within a same irradiation volume, rather than trying to spare the healthy cells with a risk of keeping tumoural cells active, In a preferred embodiment, the TPS comprises the delivery to an entirety of the uncertain zone (3z) at the end of N fractions of a total uncertain dose (DuTi). If healthy cells are to be spared, then the maximum value of DuTi is defined by the preservation of the healthy cells. In these conditions, we can stipulate that the total uncertain doses DuTi is equivalent to the total healthy dose (DhTi), and the following conditions apply to healthy cells comprised in an uncertain zone or any irradiated volume comprising healthy cells exposed to a same irradiation dose as the tumoural cells comprised within the irradiated volume, such as for example, but not restricted to, an island of healthy cells enclosed within the peripheral surface, as illustrated in Figures 2(a) and 2(b). In this case, it is preferred that, as illustrated in Figure 7,
- the total healthy dose (DhTi = DchTi + DhhTi) is larger than the minimum target dose (DmtT0), and lower than the maximum high rate healthy dose (DhhT0i) (i.e., DmtT0 < DhTi < DhhT0i), wherein DchTi and DhhTi are the conventional and high rate healthy doses deposited at CDR and HDR, respectively, cumulated over the N fractions j and
- a total equivalent healthy dose (DmhTi = DchTi + α DhhTi) delivered at the end of the N fractions is lower than the maximum conventional healthy dose (DchT0i) of the healthy tissue (3hi) present in the uncertain zone (i.e., DmhTi < DchT0i), and
- an equivalent healthy fraction dose (Dmhij = Dchij + α Dhhij) delivered at each fraction j is lower than the maximum conventional healthy fraction dose (DchT0ij) of the healthy tissue (3hi) present in the uncertain zone, (i.e., DmhTi < DchT0i).

### MULTI-STEP TREATMENT PLAN

In an embodiment of the invention, one or more fractions of the treatment plan include a first step of delivering a conventional dose at a conventional dose deposition rate (CDR) with a first charged particles beam or group of charged particles beams parallel to a first direction each formed by various beamlets. Referring to Figure 1(a), this would correspond to the delivery in the first step of all the beamlets labelled "Dchij". The conventional dose is such that,
- a conventional target fraction dose (Dctj) is delivered at each or some of the one or more fractions to at least 90%; preferably at least 95%, more preferably at least 99% of the tumoral cells of the target tissue (3t), and
- a conventional healthy fraction dose (Dchi) lower than the maximum conventional healthy fraction dose (Dch0ij) is delivered at each or some of the one or more fractions to the healthy tissues (3hi) surrounding and enclosed within the peripheral surface (i.e., Dchij < Dch0ij).

This first (conventional) step is followed or preceded by a second step of delivering a high rate dose at an ultra-high dose deposition rate (HDR) with a second charged particles beam or group of charged particles beams parallel to a second direction same as or different from the first direction. Referring to Figure 1(a), this would correspond to the delivery in the second step of the beamlets labelled "Dhhij". The high rate dose is such that,
- a high rate target fraction dose (Dhtj) is delivered at each or some of the one or more fractions to the target tissue (3t) such that 100% of the tumoral cells of the target tissue (3t) have received a fraction dose at conventional and / or at ultra-high rates (CDR, HDR), and
- a high rate healthy fraction dose (Dhhij) is delivered at each or some of the one or more fractions to the healthy tissues (3hi) surrounding and within the peripheral surface, such that the equivalent healthy fraction dose (Dmhij = Dchij + α Dhhij) delivered at both conventional and ultra-high rates (CDR, HDR) to all healthy cells, does not exceed the maximum conventional healthy fraction dose (Dch0ij) (i.e. Dmhij ≤ Dch0ij).

The first step of conventional irradiation is like painting an area enclosed within a perimeter with a large brush, followed by painting the edges of the area with a thinner brush, to ensure that the area adjacent to and within the perimeter is fully coloured, whilst no colour is visible outside the perimeter. First and second steps can be carried out in any order.

To ensure that in a fraction j the healthy cells do not receive a healthy fraction dose (Dhii) cumulated over the first and second steps, which exceeds the maximum healthy fraction dose (Dh0ij), the treatment plan can ensure that in a same fraction the second charged particles beam or group of charged particles beams does not overlap with the first charged particles beam or group of charged particles beams in any healthy tissue (3hi), surrounding or within the peripheral surface.

### SINGLE-STEP TREATMENT PLAN

In an alternative embodiment of the invention, one or more fractions of the treatment plan include a single charged particles beam (PB) or group of beams parallel to a first direction formed by beamlets, such that,
- a conventional target fraction dose (Dctj) is delivered to at least 90%, preferably at least 95%, more preferably at least 99% of the tumoral cells of the target tissue (3t),
- a conventional healthy fraction dose (Dchij) lower than the maximum conventional healthy fraction dose (Dch0ij) is delivered to the healthy tissues (3hi) surrounding and enclosed within the peripheral surface (i.e., Dchij < Dch0ij),
- a high rate target fraction dose (Dhtj) is delivered at each or some of the one or more fractions to the target tissue (3t) such that 100% of the tumoral cells of the target tissue (3t) have received a fraction dose at conventional and / or at ultra-high rates (CDR, HDR), and
- a high rate healthy fraction dose (Dhhij) is delivered at each or some of the one or more fractions to the healthy tissues (3hi) surrounding and within the peripheral surface, such that the equivalent healthy fraction dose (Dmhij = Dchij + α Dhhij) delivered at both conventional and ultra-high rates (CDR, HDR) to all healthy cells, does not exceed the maximum conventional healthy fraction dose (Dch0ij) (i.e. Dmhij ≤ Dch0ij).

The difference of the present single-step treatment plan with the multi-step treatment plan discussed supra, is that all beamlets of a fraction j are delivered within a single irradiation step instead of two steps for the multi-step treatment plan. Referring to Figure 1(a), this means that in a single step, the beamlets labelled "Dchij" and the beamlets labelled "Dhhij" are all delivered within the same irradiation beam which is modulated to vary the doses deposited as a function of penetration depth (z).

Figure 3 shows N fractions, each fraction comprising deposition of a conventional fraction dose (Dchij) (shaded columns) and of a high rate fraction (Dhhij) (white columns). The HDR-proportion, xH = Dhhij / (Dchij + Dhhij) can vary substantially from one fraction to the other. The respective conventional and high rate fraction doses must fulfil the following criteria,
- the total target dose (DmtT) cumulated over the N fractions (DmtT = Σ Dmtj = DctT + DhtT = Σ (Dctj + Dhtj)) must be larger than the predefined minimum target dose (DmtT0), (i.e., DmtT > DmtT0), wherein DctT and DhtT are the conventional and high rate target doses deposited at CDR and HDR, respectively, cumulated over the N fractions j, and
- the total equivalent healthy dose (DmhTi) cumulated over the N fractions (DmhTi = Σ Dmhij = DchTi + α DhhTi = Σ (Dchij + α Dhhij)) must be lower than the predefined maximum conventional healthy dose (DchT0i) (i.e., DmhTi > DchT0i).

According to the multi-step treatment plan, the conventional dose (Dchij) at each fraction j (cf., shaded columns in Figure 3) is deposited in the first step, followed by the second step of depositing the high rate dose (Dhhij). According to the single-step treatment plan, all beamlets depositing doses at both CDR and HDR are sent in a single irradiation, with the properties (including deposition rate) of the successive beamlets changing with depth (z) of the corresponding Bragg peaks.

### SUMMARY

A treatment plan for treatment with charged particles beams, preferably with proton beams, of a target tissue comprising tumoural cells must fulfil predefined clinical criteria for ensuring that at the end of the treatment, the tumoural cells are killed, whilst sparing the healthy cells adjacent to the tumoural cells. The criteria include
(1) Killing of tumoural cells
   (C1) the total target dose (DmtT) delivered to the whole volume comprised within the peripheral surface must exceed the minimum target dose (DmtT0), i.e., DmtT = Σ Dmtj ≥ DmtT0,
(2) Sparing of healthy cells
   (C2.1) the healthy fraction dose (Dhij) deposited in one fraction j cannot exceed the maximum healthy fraction dose (Dh0ij), i.e., Dhij = Dchij + Dhhij ≤ Dh0ij, and
   (C2.2) the healthy dose cumulated over the N fractions j (DhTi) cannot exceed the maximum healthy dose cumulated over the N fractions j (DhT0i), i.e., DhTi = Σ Dhij = Σ (Dchij + Dhhij) ≤ DhT0i.

If healthy cells are adjacent to tumoural cells, the healthy cells are likely to receive a same dose as the tumoural cells. If the dose received is sufficient for killing the tumoural cells, it is likely that it is also sufficient for killing the healthy cells, which is of course undesirable. The present invention takes advantage of the FLASH effect observed at ultra-high dose deposition rates (HDR) by combining in the treatment plan conventional irradiation at CDR and FLASH-irradiation of specific volumes at HDR.

Criterion (C1) is fulfilled by ensuring that the total target dose DmtT = DctT + DhtT is greater than the minimum target dose (DmtT0).

Criterion (C2.1) is fulfilled by ensuring that the healthy fraction dose (Dhij = Dchij + Dhhij) is lower than the maximum healthy fraction dose (Dh0ij). This criterion is satisfied if the equivalent healthy fraction dose (Dmhij = Dchij + α Dhhij) does not exceed the maximum conventional healthy fraction dose (Dch0ij), i.e., Dmhij = Dchij + α Dhhij ≤ Dch0ij. The equivalent healthy fraction dose (Dmhij) is the dose deposited at least partly at HDR yielding a same value of complication probability (NTCP0i) as if the same dose had been deposited at CDR only.

Criterion (C2.2) is fulfilled by ensuring that, the total healthy dose (DhTi = DchTi + DhhTi) is lower than the maximum total healthy dose (DhT0i). This criterion is satisfied if the equivalent healthy dose (DmhTi = DchTi + α Dhhij) does not exceed the maximum conventional healthy fraction dose (DchT0i, i.e., Dmhij = Dchij + α DhhTi ≤ DchT0i. The equivalent total healthy dose (DmhTi) is the cumulated dose deposited at least partly at HDR over the N fractions, yielding a same value of complication probability (NTCP0i) as if the same dose had been deposited at CDR only.

With the above treatment plan, if the healthy fraction dose (Dhij) deposited onto the healthy cells in a fraction j is larger than both the minimum target dose (DmtT0) required for killing the tumoural cells and the healthy cells comprised within the volume radiated in fraction j, the apparently inescapable trap is opened by combining deposition at CDR with deposition at HDR to profit of the FLASH effect. The trap is open even in the worst-case scenario wherein both tumoural and healthy cells receive a same radiation dose (Dmtij = Dhij), thus fulfilling both Criteria (C1) and (C2.1). The same conclusion applies to the full treatment at the end of N fractions, such that if the total healthy fraction (DhTi) deposited onto the healthy cells after N fractions is deposited at least partly at HDR, it can be larger than the minimum target dose (DmtT0) required for killing the tumoural cells within the peripheral surface, and lower than the maximum total healthy dose (DhT0i) required for sparing the healthy tissues (3hi). Here again the apparently inescapable trap is opened even in the worst-case scenario wherein both tumoural and healthy cells receive a same radiation dose, thus fulfilling both Criteria (C1) and (C2.2). The cases wherein Dhij = Dmtj and, after N fractions, DhTi = DmTt is referred to as "worst case scenario," because in most cases, if the healthy cells are outside the peripheral surface, the treatment plan attempts to reduce the dose (Dhij) delivered to the healthy cells and to maximize the dose (Dmtj) delivered to the tumoural cells, so that Dhij < Dmtj and, after N fractions, DhTi < DmTt.

The treatment plan generated by the TPS of the present invention and preferred embodiments thereof can be implemented in a method for treating with charged particles beams, the method not being part of the claimed invention, preferably with proton beams, a target tissue (3t) comprising tumoral cells enclosed within a peripheral surface, which is surrounded by healthy cells and/or encloses also healthy cells, wherein the healthy cells form healthy tissues (3hi), wherein the subscript i designates the type of healthy tissues (3hi), wherein the method consists of N fractions of irradiation, with N ≥ 1, and comprises fulfilling the following criteria,
(C1) at the end of the N fractions, all tumoral cells of the target tissue (3t) must have received a total target dose (DmtT) equal to the sum of target fraction doses (Dmtj) received at each fraction (i.e., DmtT = Σ Dmtj), which is at least equal to a minimum target dose (DmtT0) for killing the tumoral cells, i.e., DmtT = Σ Dmtj ≥ DmtT0,
(C2) at the end of the N fractions, all healthy cells (3hi) of the healthy tissues surrounding or enclosed within the peripheral surface must have received a total healthy dose (DhTi) equal to the sum of the healthy fraction doses (Dhij) received at each fraction j (i.e., DhTi = Σ Dhij), such that,
   (C2.1) the healthy fraction dose (Dhij) received at each fraction j does not exceed a predefined threshold for preserving the healthy cells at the end of the fraction j, and
   (C2.2) the total healthy doses (DhTi) received and cumulated at the end of the N fractions does not exceed a predefined threshold for preserving the healthy cells at the end of the treatment of N fractions,
**wherein**
- the method combines N fractions including,
   o irradiation of volumes including tumoral cells and/or healthy cells at a conventional dose rate (CDR), defined as a dose rate, CDR < 1 Gy / s, and / or
   o irradiation of volumes including tumoral cells and/or healthy cells at an ultra-high dose rate (HDR), defined as a dose rate, HDR ≥ 1 Gy / s, wherein
- the total target dose (DmtT) delivered to and cumulated in a volume of the target tissue (3t) at the end of N fractions is defined to fulfill criterion (C1) as a sum over the N fractions j, DmtT = Σ Dmtj = Σ (Dctj + Dhtj) ≥ DmtT0, of target fraction doses (Dmtj) delivered to the target tissues (3t) at each fraction, wherein the target fraction dose (Dmtj) delivered during a fraction j is defined as a sum of,
   o conventional target doses (Dctj) delivered to that volume at conventional dose rates (CDR) during the fraction j, and of
   o high rate target doses (Dhtj) delivered to that volume at ultra-high dose rates (HDR) during the fraction j,
and **wherein,**
- the healthy fraction dose (Dhij) delivered to a volume of healthy cells of a healthy tissue (3i) during a fraction j is equal to a sum, Dhij = Dchij + Dhhij, of
   ∘ a conventional healthy fraction dose (Dchij) delivered to that volume at a conventional dose rate (CDR), and of
   ∘ a high rate healthy fraction dose (Dhhij) delivered to that volume at ultra-high dose rate (HDR),
- an equivalent healthy fraction dose (Dmhij) defined as a sum Dmhij = (Dchij + α Dhhij), of
   o the conventional healthy fraction dose (Dchij) and
   o a product of an equivalence coefficient (α) and of the high rate healthy fraction dose (Dhhij),
      does not exceed a maximum equivalent healthy fraction dose (Dmh0ij), for preserving the healthy cells after each fraction j (i.e., Dmhij ≤ Dmh0ij), and thus fulfilling criterion (C2.1), wherein the maximum equivalent healthy fraction dose (Dmh0ij) is smaller than or equal to a maximum healthy conventional fraction dose (Dch0ij) corresponding to a dose deposited onto a healthy cell at a conventional dose rate (CDR) only and yielding a predefined probability of complication (NTCP0i) of the healthy cell (3hi) (i.e., Dmh0ij ≤ Dch0ij)
- a total equivalent healthy dose (DmhTi) delivered to and cumulated in a volume of a healthy tissue (3hi) at the end of N fractions is defined as a sum over the N fractions j, DmhTi = Σ Dmhij = Σ (Dchij + α Dhhij) with α < 1, preferably α > 0.5, of the equivalent healthy
   fraction doses (Dmhij), wherein the total equivalent healthy dose (DmhTi) does not exceed a maximum equivalent healthy dose (DmhT0i), for preserving the healthy cells at the end of the N fractions (i.e., DmhTi ≤ DmhT0i ), and thus fulfilling criterion (C2.2), wherein the maximum equivalent healthy dose (DmhT0i) is smaller than a maximum healthy conventional dose (DchT0i) corresponding to a dose deposited onto a healthy cell at a conventional dose rate (CDR) only and yielding a predefined probability of complication (NTCP0i) of the healthy cell (3hi) (i.e., DmhT0i < DchT0i)

wherein the equivalent healthy fraction dose (Dmhij) and the total equivalent healthy dose (DmhTi) are equivalent doses yielding a same or lower probability of complication of a healthy cell (3hi) (NTCP0) as if the healthy fraction dose (Dhij) and the total healthy dose (DhTi) had, respectively, been deposited onto the healthy cell at a conventional dose rate (CDR) only.

| **REF** | **DESCRIPTION** |
|---|---|
| **3hi** | Healthy tissue i |
| **3t** | Target tissue |
| **3z** | Uncertain zone |
| **D** | Dose |
| **Dch0ij** | Maximum conventional healthy fraction dose |
| DchT0i | Maximum conventional healthy dose cumulated over the N fractions j |
| Dchi1 | Conventional healthy fraction dose of a fraction j = 1 |
| **Dchij** | Conventional healthy fraction dose |
| DchTi | Conventional healthy dose cumulated over the N fractions j |
| DctT | Conventional target dose |
| Dctj | Conventional target fraction dose |
| Dh0ij | Maximum healthy fraction dose |
| Dhi0ij(1) | Maximum healthy fraction dose, (α = const) |
| Dhi0ij(2) | Maximum healthy fraction dose, (α = f(xH) is linear) |
| Dhi0ij(3) | Maximum healthy fraction dose, (α = f(xH) is parabolic) |
| Dhi0ij(4) | Maximum healthy fraction dose, (α = f(xH) is parabolic) |
| Dhhi1 | High rate healthy fraction dose of a fraction j = 1 |
| Dhhij | High rate healthy fraction dose |
| Dhh0ij | maximum high rate healthy fraction dose |
| DhhT0i | Maximum high rate healthy dose |
| DhhTi | High rate healthy dose |
| Dhi1 | Healthy fraction dose of a fraction j = 1 |
| Dhij | Dhij = Dchij + Dhhij is the healthy fraction dose |
| DhTi | DhTi = ∑ Dhij = ∑ (Dchij + Dhhij) is the total healthy dose cumulated over the N fractions j |
| Dhtj | High rate target fraction dose |
| DhtT | High rate target dose |
| DhT0i | Maximum total healthy dose cumulated over the N fractions j |
| Dmh0ij | Maximum equivalent healthy fraction dose |
| Dmhi1 | equivalent healthy fraction dose of a fraction j = 1 |
| Dmhij | Dmhij = Dchij+ α Dhhij is the equivalent healthy fraction dose |
| DmhTi | DmhTi = ∑ Dmhij = ∑ (Dchij + α Dhhij) is the total equivalent healthy dose cumulated over the N fractions j |
| Dmt0j | Minimum target fraction dose |
| Dmtj | Dmtj = Dctj + Dhtj is the target fraction dose |
| DmtT | DmtT = DctT + DhtT is the Total target dose |
| DmtT0 | Minimum target dose |
| DR | Dose deposition rate (or deposition rate) |
| k | CDR / HDR proportionality factor at ultra-high dose deposition rates, (k = 1/α) |
| CDR | conventional dose deposition rate |
| HDR | Ultra-high dose deposition rate |
| NTCP | Normal Tissue Complication Probabiliy |
| NTCP0i | Maximum NTCP value for a healthy tissue (3hi) |
| PB | Proton beam |
| S | Skin |
| SOBP | Sum of Bragg Peaks |
| x, y | Vectors defining plane normal to beam at depth (z) |
| xH | HDR-proportion = Dhhij / (Dchij + Dhhij) |
| XR | X-ray |
| Z | Penetration depth |
| Z0 | Z = 0 (skin) |
| Zn | Deepest portion of the peripheral surface |
| α | Equivalence coefficient (Dmhij = Dchij + α Dhhij) |

## Claims

1. Treatment planning system (TPS) for generating a plan for treatment with charged particles beams, preferably with proton beams, of a target tissue (3t) comprising tumoral cells enclosed within a peripheral surface, which is surrounded by healthy cells and/or encloses also healthy cells, wherein the healthy cells form healthy tissues (3hi), wherein the subscript i designates the type of healthy tissues (3hi), wherein the plan consists of N fractions of irradiation, with N ≥ 1, and the plan comprises fulfilling the following criteria,
(C1) at the end of the N fractions, all tumoral cells of the target tissue (3t) must have received a total target dose (DmtT) equal to the sum of target fraction doses (Dmtj) received at each fraction (i.e., DmtT = Σ Dmtj), which is at least equal to a minimum target dose (DmtT0) for killing the tumoral cells, i.e., DmtT = Σ Dmtj ≥ DmtT0,
(C2) at the end of the N fractions, all healthy cells (3hi) of the healthy tissues surrounding or enclosed within the peripheral surface must have received a total healthy dose (DhTi) equal to the sum of the healthy fraction doses (Dhij) received at each fraction j (i.e., DhTi = Σ Dhij), such that,
(C2.1) the healthy fraction dose (Dhij) received at each fraction j does not exceed a predefined threshold for preserving the healthy cells at the end of the fraction j, and
(C2.2) the total healthy doses (DhTi) received and cumulated at the end of the N fractions does not exceed a predefined threshold for preserving the healthy cells at the end of the treatment of N fractions,
wherein the TPS comprises a computer configured for generating the plan by combining N fractions including,
o irradiation of volumes including tumoral cells and/or healthy cells at a conventional dose deposition rate (CDR), defined as a dose rate, CDR < 1 Gy / s, and / or
o irradiation of volumes including tumoral cells and/or healthy cells at an ultra-high dose deposition rate (HDR), defined as a dose rate, HDR ≥ 1 Gy / s,
such that the total target dose (DmtT) delivered to and cumulated in a volume of the target tissue (3t) at the end of N fractions is defined to fulfill criterion (C1) as a sum over the N fractions j, DmtT = Σ Dmtj = Σ (Dctj + Dhtj) ≥ DmtT0, of target fraction doses (Dmtj) delivered to the target tissues (3t) at each fraction, wherein the target fraction dose (Dmtj) delivered during a fraction j is defined as a sum of,
o conventional target doses (Dctj) delivered to that volume at conventional dose deposition rates (CDR) during the fraction j, and of
o high rate target doses (Dhtj) delivered to that volume at ultra-high dose deposition rates (HDR) during the fraction j,
wherein,
• the healthy fraction dose (Dhij) delivered to a volume of healthy cells of a healthy tissue (3i) during a fraction j is equal to a sum, Dhij = Dchij + Dhhij, of
o a conventional healthy fraction dose (Dchij) delivered to that volume at a conventional dose deposition rate (CDR), and of
o a high rate healthy fraction dose (Dhhij) delivered to that volume at ultra-high dose deposition rate (HDR),
• an equivalent healthy fraction dose (Dmhij) defined as a sum Dmhij = (Dchij + α Dhhij), of
o the conventional healthy fraction dose (Dchij) and
o a product of an equivalence coefficient (a) and of the high rate healthy fraction dose (Dhhij),
does not exceed a maximum equivalent healthy fraction dose (Dmh0ij), for preserving the healthy cells after each fraction j (i.e., Dmhij ≤ Dmh0ij), and thus fulfilling criterion (C2.1), wherein the maximum equivalent healthy fraction dose (Dmh0ij) is smaller than or equal to a maximum healthy conventional fraction dose (Dch0ij) corresponding to a dose deposited onto a healthy cell at a conventional dose deposition rate (CDR) only and yielding a predefined probability of complication (NTCP0i) of the healthy cell (3hi) (i.e., Dmh0ij ≤ Dch0ij)
• a total equivalent healthy dose (DmhTi) delivered to and cumulated in a volume of a healthy tissue (3hi) at the end of N fractions is defined as a sum over the N fractions j, DmhTi = Σ Dmhij = Σ (Dchij + α Dhhij) with α < 1, preferably α > 0.5, of the equivalent healthy fraction doses (Dmhij), wherein the total equivalent healthy dose (DmhTi) does not exceed a maximum equivalent healthy dose (DmhT0i), for preserving the healthy cells at the end of the N fractions (i.e., DmhTi ≤ DmhT0i ), and thus fulfilling criterion (C2.2),
wherein the equivalent healthy fraction dose (Dmhij) and the total equivalent healthy dose (DmhTi) are equivalent doses yielding a same or lower probability of complication of a healthy cell (3hi) (NTCP0i) as if the healthy fraction dose (Dhij) and the total healthy dose (DhTi) had, respectively, been deposited onto the healthy cell at a conventional dose deposition rate (CDR) only,
wherein the maximum equivalent healthy dose (DmhT0i) is smaller than a maximum healthy conventional dose (DchT0i) corresponding to a dose deposited onto a healthy cell at a conventional dose deposition rate (CDR) only and yielding a predefined probability of complication (NTCP0i) of the healthy cell (3hi) (i.e., DmhT0i < DchT0i),

2. TPS according to claim 1, wherein if a volume of a healthy tissue (3i) is irradiated during a fraction j,
• at a conventional dose deposition rate (CDR) only, then Dmhij = Dchij ≤ Dch0ij for that volume at that fraction, or
• at an ultra-high rate (HDR) only, then Dmhij = α Dhhij ≤ Dch0ij and Dhhij ≤ Dhh0ij for that volume at that fraction, wherein Dhh0ij is the maximum high rate healthy fraction dose delivered at HDR only at a single fraction j, not to be exceeded for preserving the healthy cells of the healthy tissue (3i), or
• at both conventional dose deposition rate (CDR) and at ultra-high dose deposition rate (HDR), Dmhij = Dchij + α Dhhij ≤ Dch0ij and Dhij = Dchij + Dhhij < Dhh0ij.

3. TPS according to claim 2, wherein the equivalence coefficient α either,
• Is a constant, preferably equal to 1 / k, wherein k = 1.2 to 1.5 (i.e., α = 1 / k, with k = 1.2 to 1.5), and preferably, a < 0.9, more preferably α < 0.85, most preferably α < 0.7, or
• depends on a HDR-proportion, xH = Dhhij / (Dchij + Dhhij) of doses deposited at ultra-high rate (HDR), and varies between 1 / k for xH = 1 (i.e., at HDR only), and 1 for xH = 0 (i.e., at CDR only).

4. TPS according to claim 2 or 3, wherein
• Dch0ij and DchT0i are predefined by a medical practitioner or defined in the literature, and
• Dhh0i and DhhT0i are either
o predefined by a medical practitioner or defined in the literature for given values of the HDR, or
o respectively defined as Dhh0ij = k Dch0i and Dhh0Ti = k DchT0i, for given values of the HDR, wherein k is comprised between 1.2 and 1.5 (i.e., k = 1.2 to 1.5), or
o defined by a pre-established curve of a Normal Tissue Complication Probability (NTCP) for the healthy tissue as a function of dose and dose rate, and wherein Dhh0ij and DhhT0i are defined as the doses delivered at HDR only having same NTCP as the doses Dch0ij and as DchT0i, respectively, delivered at CDR only..

5. Treatment planning system according to any one of claims 2 to 4, wherein all healthy tissues located upstream from the peripheral surface are irradiated at conventional dose deposition rates only during the N fractions, and wherein,
• the healthy fraction dose (Dhij) deposited in said healthy tissues at each fraction j does not exceed the maximum healthy conventional fraction dose (Dch0ij), and
• the total healthy dose (DhTi) deposited in said healthy tissues at the end of the N fractions does not exceed the maximum healthy conventional dose (Dch0Ti), wherein a healthy cell is located upstream of the peripheral surface when a charged particles beam defined in the TPS traverses said healthy cell before penetrating into the peripheral surface.

6. Treatment planning system according to any one of claims 2 to 5, wherein all healthy tissues located downstream of the peripheral surface are irradiated at ultra-high dose deposition rates only, and wherein,
• the healthy fraction dose (Dhij) deposited in said healthy tissues at each fraction j does not exceed the maximum high rate healthy fraction dose (Dhh0ij), and
• the total healthy dose (DhTi) deposited in said healthy tissues at the end of the N fractions does not exceed the maximum healthy conventional dose (Dch0Ti),
wherein a healthy cell is located downstream of the peripheral surface when a charged particles beam traverses said healthy cell only after exiting the peripheral surface.

7. Treatment planning system according to any one of the preceding claims, wherein one or more of the fractions of the plan include the following steps carried out in any sequential order,
• a first step of delivering a conventional dose at a conventional dose deposition rate (CDR) with a first charged particles beam or group of charged particles beams parallel to a first direction each formed by various beamlets, such that,
o a conventional target fraction dose (Dctj) is delivered at each or some of the one or more fractions to at least 90%; preferably at least 95%, more preferably at least 99% of the tumoral cells of the target tissue (3t), and
o a conventional healthy fraction dose (Dchi) lower than the maximum healthy conventional fraction dose (Dch0ij) is delivered at each or some of the one or more fractions to the healthy tissues (3hi) surrounding and enclosed within the peripheral surface (i.e., Dchij < Dch0ij), and
• a second step, following the first step, of delivering a high rate dose at an ultra-high dose deposition rate (HDR) with a second charged particles beam or group of charged particles beams parallel to a second direction same as or different from the first direction, such that,
o a high rate target fraction dose (Dhtj) is delivered at each or some of the one or more fractions to the target tissue (3t) such that 100% of the tumoral cells of the target tissue (3t) have received a fraction dose at conventional and / or at ultra-high rates (CDR, HDR), and
o a high rate healthy fraction dose (Dhhij) is delivered at each or some of the one or more fractions to the healthy tissues (3hi) surrounding and within the peripheral surface, such that the equivalent healthy fraction dose (Dmhij = Dchij + α Dhhij) delivered at both conventional and ultra-high rates (CDR, HDR) to all healthy cells, does not exceed the maximum conventional healthy fraction dose (Dmh0ij) (i.e. Dmhij ≤ Dch0ij).

8. Treatment planning system according to the preceding claim 7, wherein the second charged particles beam or group of charged particles beams does not overlap with the first charged particles beam or group of charged particles beams in any healthy tissue (3hi), surrounding or within the peripheral surface.

9. Treatment planning system according to any one of claims 1 to 4, wherein the plan includes a single charged particles beam (PB) or group of beams parallel to a first direction formed by beamlets, such that,
• a conventional target fraction dose (Dctj) is delivered to at least 90%, preferably at least 95%, more preferably at least 99% of the tumoral cells of the target tissue (3t),
• a conventional healthy fraction dose (Dchij) lower than the maximum healthy conventional fraction dose (Dch0ij) is delivered to the healthy tissues (3hi) surrounding and enclosed within the peripheral surface (i.e., Dchij < Dch0ij),
• a high rate target fraction dose (Dhtj) is delivered at each or some of the one or more fractions to the target tissue (3t) such that 100% of the tumoral cells of the target tissue (3t) have received a fraction dose at conventional and / or at ultra-high rates (CDR, HDR), and
• a high rate healthy fraction dose (Dhhij) is delivered at each or some of the one or more fractions to the healthy tissues (3hi) surrounding and within the peripheral surface, such that the equivalent healthy fraction dose (Dmhij = Dchij + α Dhhij) delivered at both conventional and ultra-high rates (CDR, HDR) to all healthy cells, does not exceed the maximum conventional healthy fraction dose (Dch0ij) (i.e. Dmhij ≤ Dch0ij).

10. Treatment planning system according to any one of the preceding claims 2 to 9, wherein the peripheral surface comprises an uncertain volume defining an uncertain zone (3z), defined as a zone comprising both tumoural and healthy cells intermixed at different ratios, and wherein the TPS comprises the delivery to an entirety of the uncertain volume at the end of N fractions of a total healthy dose (DhTi) such that,
• the total healthy dose (DhTi = DchTi + DhhTi) is larger than the minimum target dose (DmtT0), and lower than the maximum high rate healthy dose (DhhT0i) (i.e., DmtT0 < DhTi < DhhT0i), and
• a total equivalent healthy dose (DmhTi = DchTi + α DhhTi) delivered at the end of the N fractions is lower than the maximum healthy conventional dose (DchT0i) of the healthy tissue (3hi) present in the uncertain zone (i.e., DmhTi < DchT0i), and
• an equivalent healthy fraction dose (Dmhij = Dchij + α Dhhij) delivered at each fraction j is lower than the maximum healthy conventional fraction dose (DchT0ij) of the healthy tissue (3hi) present in the uncertain zone, (i.e., DmhTi < DchT0i).

11. Treatment planning system according to any one of the preceding claims, wherein the maximum healthy conventional dose (DchT0i) at the end of the N fractions is defined as follows for a selection of healthy tissues (3hi),
| Healthy tissue (3hi) | DchT0i (Gy) |
|---|---|
| brain | 60 |
| heart | 50 |
| larynx | 80 |
| Liver | 40 |
| Lung | 25 |
| Stomach | 65 |
| Eye lens | 18 |

12. Treatment planning system according to the preceding claim, wherein the maximum high rate healthy dose (DhhT0i) at the end of the N fractions is defined as follows for the selection of healthy tissues (3hi),
| Healthy tissue (3hi) | DhhT0i (Gy) |
|---|---|
| brain | 90 |
| heart | 75 |
| larynx | 120 |
| Liver | 60 |
| Lung | 38 |
| Stomach | 98 |
| Eye lens | 27 |

13. Treatment planning system according to any one of the preceding claims, wherein the charged particle beam is a proton beam delivered in single- or double-scattering mode, or by pencil beam scanning (PBS).

## Patentansprüche

1. Behandlungsplanungssystem (TPS) zum Erzeugen eines Plans für eine Behandlung mit Strahlen geladener Partikel, vorzugsweise mit Protonenstrahlen, eines Zielgewebes (3t), das Tumorzellen umfasst, die in einer peripheren Oberfläche eingeschlossen sind, die von gesunden Zellen umgeben ist und/oder auch gesunde Zellen einschließt, wobei die gesunden Zellen gesundes Gewebe (3hi) ausbilden, wobei der Index i den Typ von gesunden Geweben (3hi) bezeichnet, wobei der Plan aus N Teilbestrahlungen besteht, wobei N ≥ 1 ist, und der Plan Erfüllen der folgenden Kriterien umfasst,
(C1) am Ende der N Teile müssen alle Tumorzellen des Zielgewebes (3t) eine Gesamtzieldosis (DmtT) gleich der Summe von Teilzieldosen (Dmtj) empfangen haben, die mit jedem Teil empfangen wurden (d. h. DmtT = Σ Dmtj), die mindestens gleich einer minimalen Zieldosis (DmtT0) zum Abtöten der Tumorzellen ist, d. h. DmtT = Σ Dmtj ≥ DmtT0,
(C2) am Ende der N Teile müssen alle gesunden Zellen (3hi) des umgebenden gesunden Gewebes, oder das in der peripheren Oberfläche eingeschlossen ist, eine Gesamtgesunddosis (DhTi) gleich der Summe der Teilgesunddosen (Dhij) empfangen haben, die mit jedem Teil j empfangen wurden (d. h. DhTi = Σ Dhij), so dass,
(C2.1) die Teilgesunddosis (Dhij), die mit jedem Teil j empfangen wird, einen vordefinierten Schwellenwert zum Erhalten der gesunden Zellen am Ende des Teils j nicht übersteigt und
(C2.2) die empfangenen und am Ende der N Teile kumulierten Gesamtgesunddosen (DhTi) einen vordefinierten Schwellenwert zum Erhalten der gesunden Zellen am Ende der Behandlung mit N Teilen nicht übersteigen,
wobei das TPS einen Computer umfasst, der zum Erzeugen des Plans durch Kombinieren von N Teilen eingerichtet ist, der Folgendes umfasst,
o Bestrahlung von Volumen, die Tumorzellen und/oder gesunde Zellen enthalten, mit einer konventionellen Dosisanreicherungsrate (CDR), die als eine Dosisrate, CDR < 1 Gy / s, definiert ist, und / oder
o Bestrahlung von Volumen, die Tumorzellen und/oder gesunde Zellen enthalten, mit einer ultrahohen Dosisanreicherungsrate (CDR), die als eine Dosisrate, HDR ≥ 1 Gy / s, definiert ist,
so dass die Gesamtzieldosis (DmtT), die in ein Volumen des Zielgewebes (3t) am Ende von N Teilen geliefert wird und dort kumuliert, definiert ist, Kriterium (C1) als eine Summe über die N Teile j, DmtT = Σ Dmtj = Σ (Dctj + Dhtj) ≥ DmtT0, der Teilzieldosen (Dmtj), die an die Zielgewebe (3t) mit jedem Teil geliefert werden, erfüllen, wobei die Teilzieldosis (Dmtj), die während eines Teils j geliefert wird, als eine Summe definiert ist, bestehend aus
o konventionellen Zieldosen (Dctj), die in dieses Volumen mit konventionellen Dosisanreicherungsraten (CDR) während des Teils j geliefert werden, und aus
o Zieldosen mit hoher Rate (Dhtj), die in dieses Volumen mit ultrahohen Dosisanreicherungsraten (HDR) während des Teils j geliefert werden,
wobei,
• die Teilgesunddosis (Dhij), die in ein Volumen aus gesunden Zellen eines gesunden Gewebes (3i) während eines Teils j geliefert wird, gleich einer Summe, Dhij = Dchij + Dhhij, ist, bestehend aus
o einer konventionellen Teilgesunddosis (Dchij), die in dieses Volumen mit einer konventionellen Dosisanreicherungsrate (CDR) geliefert wird, und aus
o einer Teilgesunddosis mit hoher Rate (Dhhij), die in dieses Volumen mit ultrahoher Dosisanreicherungsrate (HDR) geliefert wird,
• eine äquivalente Teilgesunddosis (Dmhij), die als eine Summe Dmhij = (Dchij + α Dhhij) definiert ist, bestehend aus
o der konventionellen Teilgesunddosis (Dchij) und
o einem Produkt eines Äquivalenzkoeffizienten (α)
und der Teilgesunddosis mit hoher Rate (Dhhij),
eine maximale äquivalente Teilgesunddosis (Dmh0ij) zum Erhalten der gesunden Zellen nach jedem Teil j nicht übersteigt (d. h. Dmhij ≤ Dmh0ij) und folglich Kriterium (C2.1) erfüllt, wobei die maximale äquivalente Teilgesunddosis (Dmh0ij) kleiner oder gleich einer maximalen konventionellen Teilgesunddosis (Dch0ij) ist, die einer Dosis entspricht, die in einer gesunden Zelle nur mit einer konventionellen Dosisanreicherungsrate (CDR) angereichert wird und eine vordefinierte Wahrscheinlichkeit einer Komplikation (NTCP0i) der gesunden Zelle (3hi) ergibt (d. h. Dmh0ij ≤ Dch0ij)
• eine äquivalente Gesamtgesunddosis (DmhTi), die in ein Volumen eines gesunden Gewebes (3hi) am Ende von N Teilen geliefert und dort kumuliert wurde, als eine Summe über die N Teile j, DmhTi = Σ Dmhij = Σ (Dchij + α Dhhij), wobei α < 1 ist, vorzugsweise α > 0,5 ist, der äquivalenten Teilgesunddosen (Dmhij) definiert ist, wobei die äquivalente Gesamtgesunddosis (DmhTi) eine maximale äquivalente Gesunddosis (DmhT0i) zum Erhalten der gesunden Zellen am Ende der N Teile nicht übersteigt (d. h. DmhTi ≤ DmhT0i) und folglich Kriterium (C2.2) erfüllt,
wobei die äquivalente Teilgesunddosis (Dmhij) und die äquivalente Gesamtgesunddosis (DmhTi) äquivalente Dosen sind, die eine gleiche oder geringere Wahrscheinlichkeit einer Komplikation einer gesunden Zelle (3hi) (NTCP0i) ergeben, als wenn die Teilgesunddosis (Dhij) bzw. die Gesamtgesunddosis (DhTi) in der gesunden Zelle nur mit einer konventionellen Dosisanreicherungsrate (CDR) angereichert worden wäre,
wobei die maximale äquivalente Gesunddosis (DmhT0i) kleiner ist als eine maximale konventionelle Gesunddosis (DchT0i), die einer Dosis entspricht, die in einer gesunden Zelle nur mit einer konventionellen Dosisanreicherungsrate (CDR) angereichert wird, und eine vordefinierte Wahrscheinlichkeit einer Komplikation (NTCP0i) der gesunden Zelle (3hi) ergibt (d. h. DmhT0i < DchT0i),

2. TPS nach Anspruch 1, wobei, wenn ein Volumen eines gesunden Gewebes (3i) während eines Teils j bestrahlt wird
• mit nur einer konventionellen Dosisanreicherungsrate (CDR), dann Dmhij = Dchij ≤ Dch0ij für dieses Volumen in diesem Teil ist, oder
• mit nur einer ultrahohen Rate (HDR), dann Dmhij = α Dhhij ≤ Dch0ij und Dhhij ≤ Dhh0ij für dieses Volumen in diesem Teil ist, wobei Dhh0ij die maximale Teilgesunddosis mit hoher Rate ist, die nur mit HDR in einem einzelnen Teil j geliefert wird und die zum Erhalten der gesunden Zellen des gesunden Gewebes (3i) nicht überschritten werden darf, oder
• mit sowohl konventioneller Dosisanreicherungsrate (CDR) als auch mit ultrahoher Dosisanreicherungsrate (HDR), Dmhij = Dchij + α Dhhij ≤ Dch0ij ist und Dhij = Dchij + Dhhij < Dhh0ij ist.

3. TPS nach Anspruch 2, wobei der Äquivalenzkoeffizient α entweder,
• eine Konstante ist, vorzugsweise gleich 1 / k ist, wobei k = 1,2 bis 1,5 ist (d. h. α = 1 / k, wobei k = 1,2 bis 1,5 ist) und vorzugsweise α < 0,9 ist, besonders bevorzugt α < 0,85 ist, ganz besonders bevorzugt α < 0,7 ist, oder
• von einem HDR-Verhältnis, xH = Dhhij / (Dchij + Dhhij) von Dosen abhängt, die mit ultrahoher Rate (HDR) angereichert werden, und zwischen 1 / k für xH = 1 (d. h. nur mit HDR) und 1 für xH = 0 (d. h. nur mit CDR) variiert.

4. TPS nach Anspruch 2 oder 3, wobei
• Dch0ij und DchT0i von ärztlichem Personal vordefiniert werden oder in der Literatur definiert sind und
• Dhh0i und DhhT0i entweder
o von ärztlichem Personal vordefiniert werden oder in der Literatur für gegebene Werte der HDR definiert sind oder
o als Dhh0ij = k Dch0i bzw. Dhh0Ti = k DchT0i für gegebene Werte der HDR definiert sind, wobei k zwischen 1,2 und 1,5 liegt (d. h. k = 1,2 bis 1,5 ist), oder
o durch eine zuvor festgelegte Kurve einer normalen Wahrscheinlichkeit einer Gewebekomplikation (NTCP) für das gesunde Gewebe als eine Funktion von Dosis und Dosisrate definiert sind, und wobei Dhh0ij und DhhT0i als die Dosen, die nur mit HDR angereichert werden, mit gleicher NTCP definiert sind wie die Dosen Dch0ij bzw. DchT0i, die nur mit CDR angereichert werden.

5. Behandlungsplanungssystem nach einem der Ansprüche 2 bis 4, wobei alle gesunden Gewebe, die vor der peripheren Oberfläche angeordnet sind, nur mit konventionellen Dosisanreicherungsraten während der N Teile bestrahlt werden, und wobei,
• die Teilgesunddosis (Dhij), die in den gesunden Geweben in jedem Teil j angereichert wird, die maximale konventionelle Teilgesunddosis (Dch0ij) nicht übersteigt und
• die Gesamtgesunddosis (DhTi), die in den gesunden Geweben am Ende der N Teile angereichert wurde, die maximale konventionelle Gesunddosis (Dch0Ti) nicht übersteigt,
wobei eine gesunde Zelle vor der peripheren =Oberfläche lokalisiert ist, wenn ein Strahl geladener Partikel, der in dem TPS definiert ist, die gesunde Zelle durchquert, bevor er die periphere Oberfläche durchdringt.

6. Behandlungsplanungssystem nach einem der Ansprüche 2 bis 5, wobei alle gesunden Gewebe, die hinter der peripheren Oberfläche angeordnet sind, nur mit ultrahohen Dosisanreicherungsraten bestrahlt werden und wobei
• die Teilgesunddosis (Dhij), die in den gesunden Geweben in jedem Teil j angereichert wird, die maximale Teilgesunddosis mit hoher Rate (Dhh0ij) nicht übersteigt und
• die Gesamtgesunddosis (DhTi), die in den gesunden Geweben am Ende der N Teile angereichert wurde, die maximale konventionelle Gesunddosis (Dch0Ti) nicht übersteigt,
wobei eine gesunde Zelle hinter der peripheren Oberfläche lokalisiert ist, wenn ein Strahl geladener Partikel die gesunde Zelle nur durchquert, nachdem er die periphere Oberfläche verlassen hat.

7. Behandlungsplanungssystem nach einem der vorhergehenden Ansprüche, wobei ein oder mehrere der Teile des Plans die folgenden Schritte umfassen, die in beliebiger sequenzieller Reihenfolge ausgeführt werden,
• einen ersten Schritt des Lieferns einer konventionellen Dosis mit einer konventionellen Dosisanreicherungsrate (CDR) mit einem ersten Strahl geladener Partikel oder einer Gruppe von Strahlen geladener Partikel parallel zu einer ersten Richtung, wobei jeder durch verschiedene Strahlteile ausgebildet wird, so dass
o eine konventionelle Teilzieldosis (Dctj) mit jedem oder einigen des einen oder der mehreren Teile an mindestens 90 %; vorzugsweise mindestens 95 %, besonders bevorzugt mindestens 99 % der Tumorzellen des Zielgewebes (3t) geliefert wird und
o eine konventionelle Teilgesunddosis (Dchi), die geringer ist als die maximale konventionelle Teilgesunddosis (Dch0ij), mit jedem oder einigen des einen oder der mehreren Teile in die umgebenden gesunden Gewebe (3hi) und die in der peripheren Oberfläche eingeschlossenen geliefert wird (d. h. Dchij < Dch0ij) und
• einen zweiten Schritt, der dem ersten Schritt folgt, des Lieferns einer Dosis mit hoher Rate mit einer ultrahohen Dosisanreicherungsrate (HDR) mit einem zweiten Strahl geladener Partikel oder einer Gruppe von Strahlen geladener Partikel parallel zu einer zweiten Richtung, die gleich oder verschieden von der ersten Richtung ist, so dass
o eine Teilzieldosis mit hoher Rate (Dhtj) mit jedem oder einigen des einen oder der mehreren Teile an das Zielgewebe (3t) geliefert wird, so dass 100 % der Tumorzellen des Zielgewebes (3t) eine Teildosis mit konventioneller und / oder mit ultrahoher Rate (CDR, HDR) empfangen haben, und
o eine Teilgesunddosis mit hoher Rate (Dhhij) mit jeder oder einigen des einen oder der mehreren Teile in die umgebenden gesunden Gewebe (3hi) und in der peripheren Oberfläche geliefert wird, so dass die äquivalente Teilgesunddosis (Dmhij = Dchij + α Dhhij), die sowohl mit konventioneller als auch mit ultrahoher Rate (CDR, HDR) an alle gesunden Zellen geliefert wird, die maximale konventionelle Teilgesunddosis (Dmh0ij) nicht übersteigt (d. h. Dmhij ≤ Dch0ij).

8. Behandlungsplanungssystem nach dem vorhergehenden Anspruch 7, wobei der zweite Strahl geladener Partikel oder die zweite Gruppe von Strahlen geladener Partikel nicht den ersten Strahl geladener Partikel oder die erste Gruppe von Strahlen geladener Partikel in jedem umgebenden gesunden Gewebe (3hi) oder in der peripheren Oberfläche überlappt.

9. Behandlungsplanungssystem nach einem der Ansprüche 1 bis 4, wobei der Plan einen einzelnen Strahl geladener Partikel (PB) oder eine Gruppe von Strahlen parallel zu einer ersten Richtung umfasst, die durch Strahlteile ausgebildet ist, so dass
• eine konventionelle Teilzieldosis (Dctj) an mindestens 90 %, vorzugsweise mindestens 95 %, besonders bevorzugt mindestens 99 % der Tumorzellen des Zielgewebes (3t) geliefert wird,
• eine konventionelle Teilgesunddosis (Dchij), die geringer ist als die maximale konventionelle Teilgesunddosis (Dch0ij), an die umgebenden gesunden Gewebe (3hi) und an die in der peripheren Oberfläche eingeschlossenen geliefert wird (d. h. Dchij < Dch0ij),
• eine Teilzieldosis mit hoher Rate (Dhtj) mit jedem oder einigen des einen oder der mehreren Teile an das Zielgewebe (3t) geliefert wird, so dass 100 % der Tumorzellen des Zielgewebes (3t) eine Teildosis mit konventioneller und / oder mit ultrahoher Rate (CDR, HDR) empfangen haben, und
• eine Teilgesunddosis mit hoher Rate (Dhhij) mit jeder oder einigen des einen oder der mehreren Teile in die umgebenden gesunden Gewebe (3hi) und in der peripheren Oberfläche geliefert wird, so dass die äquivalente Teilgesunddosis (Dmhij = Dchij + α Dhhij), die sowohl mit konventioneller als auch mit ultrahoher Rate (CDR, HDR) an alle gesunden Zellen geliefert wird, die maximale konventionelle Teilgesunddosis (Dch0ij) nicht übersteigt (d. h. Dmhij ≤ Dch0ij).

10. Behandlungsplanungssystem nach einem der vorhergehenden Ansprüche 2 bis 9, wobei die periphere Oberfläche ein unbestimmtes Volumen enthält, das eine unbestimmte Zone (3z) definiert, die als eine Zone definiert ist, die sowohl Tumorzellen als auch gesunde Zellen umfasst, die mit verschiedenen Raten gemischt sind, und wobei das TPS die Lieferung in eine Gesamtheit des unbestimmten Volumens am Ende von N Teilen einer Gesamtgesunddosis (DhTi) enthält, so dass
• die Gesamtgesunddosis (DhTi = DchTi + DhhTi) größer ist als die minimale Zieldosis (DmtT0) und geringer ist als die maximale Gesunddosis mit hoher Rate (DhhT0i) (d. h. DmtT0 < DhTi < DhhT0i) und
• eine äquivalente Gesamtgesunddosis (DmhTi = DchTi + α DhhTi), die am Ende der N Teile geliefert wurde, geringer ist als die maximale konventionelle Gesunddosis (DchT0i) des gesunden Gewebes (3hi), das in der unbestimmten Zone vorhanden ist, (d. h. DmhTi < DchT0i) und
• eine äquivalente Teilgesunddosis (Dmhij = Dchij + α Dhhij), die mit jedem Teil j geliefert wird, geringer ist als die maximale konventionelle Teilgesunddosis (DchT0ij) des gesunden Gewebes (3hi), das in der unbestimmten Zone vorhanden ist, (d. h. DmhTi < DchT0i).

11. Behandlungsplanungssystem nach einem der vorhergehenden Ansprüche, wobei die maximale konventionelle Gesunddosis (DchT0i) am Ende der N Teile für eine Auswahl von gesunden Geweben (3hi) wie folgt definiert ist,
| Gesundes Gewebe (3hi) | DchT0i (Gy) |
|---|---|
| Gehirn | 60 |
| Herz | 50 |
| Kehlkopf | 80 |
| Leber | 40 |
| Lunge | 25 |
| Magen | 65 |
| Augenlinse | 18 |

12. Behandlungsplanungssystem nach dem vorhergehenden Anspruch, wobei die maximale Gesunddosis mit hoher Rate (DhhT0i) am Ende der N Teile für die Auswahl von gesunden Geweben (3hi) wie folgt definiert ist,
| Gesundes Gewebe (3hi) | DhhT0i (Gy) |
|---|---|
| Gehirn | 90 |
| Herz | 75 |
| Kehlkopf | 120 |
| Leber | 60 |
| Lunge | 38 |
| Magen | 98 |
| Augenlinse | 27 |

13. Behandlungsplanungssystem nach einem der vorhergehenden Ansprüche, wobei der Strahl geladener Partikel ein Protonenstrahl ist, der im Einfachstreuungs- oder Doppelstreuungsmodus oder durch Nadelstrahlscannen (PBS) geliefert wird.

## Revendications

1. Système de planification de traitement (TPS) destiné à générer un plan pour le traitement avec des faisceaux de particules chargées, de préférence avec des faisceaux de protons, d'un tissu cible (3t) comprenant des cellules tumorales incluses à l'intérieur d'une surface périphérique, qui est entourée par des cellules saines et/ou inclut également des cellules saines, les cellules saines formant des tissus sains (3hi), l'indice i désignant le type de tissus sains (3hi), dans lequel le plan consiste en N fractions d'irradiation, avec N ≥ 1, et le plan comprend le respect des critères suivants,
(C1) à la fin des N fractions, toutes les cellules tumorales du tissu cible (3t) doivent avoir reçu une dose cible totale (DmtT) égale à la somme de doses fractionnées cibles (Dmtj) reçues à chaque fraction (c.-à-d. DmtT = Σ Dmtj), qui est au moins égale à une dose cible minimale (DmtT0) pour tuer les cellules tumorales, c.-à-d. DmtT = Σ Dmtj ≥ DmtT0,
(C2) à la fin des N fractions, toutes les cellules saines (3hi) des tissus sains entourant ou inclus à l'intérieur de la surface périphérique doivent avoir reçu une dose saine totale (DhTi) égale à la somme de doses fractionnées saines (Dhij) reçues à chaque fraction (c.-à-d. DhTi = Σ Dhij), de telle sorte que
(C2.1) la dose fractionnée saine (Dhij) reçue à chaque fraction j ne dépasse pas un seuil prédéfini pour préserver les cellules saines à la fin de la fraction j, et
(C2.2) la dose saine totale (DhTi) reçue et cumulée à la fin des N fractions ne dépasse pas un seuil prédéfini pour préserver les cellules saines à la fin du traitement de N fractions,
dans laquelle TPS comprend un ordinateur configuré pour générer le plan en combinant N fractions comportant
o l'irradiation de volumes comportant des cellules tumorales et/ou des cellules saines à un débit de dépôt de dose conventionnel (CDR), défini comme un débit de dose CDR < 1 Gy/s, et/ou
o l'irradiation de volumes comportant des cellules tumorales et/ou des cellules saines à un débit de dépôt de dose ultrahaut (HDR), défini comme un débit de dose HDR ≥ 1 Gy/s,
de telle sorte que la dose cible totale (DmtT) délivrée à et cumulée dans un volume du tissu cible (3t) à la fin des N fractions est définie pour respecter le critère (C1) comme une somme sur les N fractions j, DmtT = Σ Dmtj = Σ(Dctj + Dhtj) ≥ DmtTo, de doses fractionnées cibles (Dmtj) délivrées au tissu cible (3t) à chaque fraction, la dose fractionnée cible (Dmtj) délivrée pendant une fraction j étant définie comme une somme
o de doses cibles conventionnelles (Dctj) délivrées à ce volume à des débits de dépôt de dose conventionnels (CDR) pendant la fraction j, et
o de doses cibles à haut débit (Dhtj) délivrées à ce volume à des débits de dépôt de dose ultrahauts (HDR) pendant la fraction j,
dans lequel
• la dose fractionnée saine (Dhij) délivrée à un volume de cellules saines d'un tissu sain (3i) pendant une fraction j est égale à une somme, Dhij = Dchij + Dhhij,
o d'une dose fractionnée saine conventionnelle (Dchij) délivrée à ce volume à un débit de dépôt de dose conventionnel (CDR), et
o d'une dose fractionnée saine à haut débit (Dhhij) délivrée à ce volume à un débit de dépôt de dose ultrahaut (HDR),
• une dose fractionnée saine équivalente (Dmhij) définie comme une somme Dmhij = (Dchij + α Dhhij)
o de la dose fractionnée saine conventionnelle (Dchij) et
o d'un produit d'un coefficient d'équivalence (a) et de la dose fractionnée saine à haut débit (Dhhij) ne dépasse pas une dose fractionnée saine équivalente maximale (Dmh0ij), pour préserver les cellules saines après chaque fraction j (c.-à-d. Dmhij ≤ Dmh0ij), et ainsi respecter le critère (C2.1), la dose fractionnée saine équivalente maximale (Dmh0ij) étant inférieure ou égale à une dose fractionnée saine conventionnelle maximale (Dch0ij) correspondant à une dosé déposée sur une cellule saine à un débit de dépôt de dose conventionnel (CDR) uniquement et générant une probabilité prédéfinie de complication (NTCP0i) de la cellule saine (3hi) (c.-à-d. Dmh0ij ≤ Dch0ij),
• une dose saine équivalente totale (DmhTi) délivrée à et cumulée dans un volume d'un tissu sain (3hi) à la fin des N fractions est définie comme une somme sur les N fractions j, DmhTi = Σ Dmhij = Σ (Dchij + α Dhhij) avec α < 1, de préférence α > 0,5, des doses fractionnées saines équivalentes (Dmhij), la dose saine équivalente totale (DmhTi) ne dépassant pas une dose saine équivalente maximale (DmhT0i), pour préserver les cellules saines à la fin des N fractions (c.-à-d. DmhTi ≤ DmhT0i), et ainsi respecter le critère (C2.2), dans lequel la dose fractionnée saine équivalente (Dmhij) et la dose saine équivalente totale (DmhTi) sont des doses équivalentes générant une probabilité identique ou inférieure de complication d'une cellule saine (3hi) (NTCP0i) par rapport à une situation où la dose fractionnée saine (Dhij) et la dose saine totale (DhTi) auraient été respectivement déposées sur la cellule saine à un débit de dépôt de dose conventionnel (CDR) uniquement,
dans lequel la dose saine équivalente maximale (DmhT0i) est inférieure à une dose saine conventionnelle maximale (DchT0i) correspondant à une dose déposée sur une cellule saine à un débit de dépôt de dose conventionnel (CDR) uniquement et générant une probabilité prédéfinie de complication (NTCP0i) de la cellule saine (3hi) (c.-à-d. DmhT0i < DchT0i).

2. TPS selon la revendication 1 dans lequel, si un volume d'un tissu sain (3i) est irradié pendant une fraction j,
• à un débit de dépôt de dose conventionnel (CDR) uniquement, alors Dmhij = Dchij ≤ Dch0ij pour ce volume à cette fraction, ou
• à un débit ultrahaut (HDR) uniquement, alors Dmhij = α Dhhij ≤ Dch0ij et Dhhij ≤ Dhh0ij pour ce volume à cette fraction, Dhh0ij étant la dose fractionnée saine à haut débit maximale délivrée à HDR uniquement au niveau d'une seule fraction j, ne devant pas être dépassée pour préserver les cellules saines du tissu sain (3i), ou
• à la fois à un débit de dépôt de dose conventionnel (CDR) et un débit de dépôt de dose ultrahaut (HDR), Dmhij = Dchij + α Dhhij ≤ Dch0ij et Dhij = Dchij + Dhhij < Dhh0ij.

3. TPS selon la revendication 2, dans lequel le coefficient d'équivalence α
• est une constante, de préférence égale à 1/k, où k = 1,2 à 1,5 (c.-à-d. α = 1/k, avec k = 1,2 à 1,5), et de préférence α < 0,9, mieux encore α < 0,85, idéalement α < 0,7, ou
• dépend d'une proportion HDR, xH = Dhhij / (Dchij + Dhhij) de doses déposées à ultrahaut débit (HDR), et varie entre 1/k pour xH = 1 (c.-à-d. à HDR uniquement) et 1 pour xH = 0 (c.-à-d. à CDR uniquement).

4. TPS selon la revendication 2 ou 3, dans lequel
• Dch0ij et DchT0i sont prédéfinies par un professionnel de santé ou définies dans la littérature, et
• Dhh0i et DhhT0i sont
oprédéfinies par un professionnel de santé ou définies dans la littérature pour des valeurs données du HDR, ou
orespectivement définies comme Dhh0ij = k Dch0i et Dhh0Ti = k DchT0i, pour des valeurs données du HDR, où k est compris entre 1,2 et 1,5 (c.-à-d. k = 1,2 à 1,5), ou
odéfinies par une courbe préétablie d'une probabilité de complication tissulaire normale (NTCP) pour le tissu sain en fonction de la dose et du débit de dose, et dans lequel Dhh0ij et DhhT0i sont définies comme les doses délivrées à HDR uniquement ayant la même NTCP que les doses Dch0ij et DchT0i, respectivement, délivrées à CDR uniquement.

5. Système de planification de traitement selon l'une quelconque des revendications 2 à 4, dans lequel tous les tissus sains situés en amont de la surface périphérique sont irradiés à des débits de dépôt de dose conventionnels uniquement pendant les N fractions, et dans lequel
• la dose fractionnée saine (Dhij) déposée dans lesdits tissus sains à chaque fraction j ne dépasse pas la dose fractionnée saine conventionnelle maximale (Dch0ij), et
• la dose saine totale (DhTi) déposée dans lesdits tissus sains à la fin des N fractions ne dépasse pas la dose saine conventionnelle maximale (Dch0Ti),
dans lequel une cellule saine est située en amont de la surface périphérique quand un faisceau de particules chargées défini dans le TPS traverse ladite cellule saine avant de pénétrer à l'intérieur de la surface périphérique.

6. Système de planification de traitement selon l'une quelconque des revendications 2 à 5, dans lequel tous les tissus sains situés en aval de la surface périphérique sont irradiés à des débits de dépôt de dose ultrahauts uniquement, et dans lequel
• la dose fractionnée saine (Dhij) déposée dans lesdits tissus sains à chaque fraction j ne dépasse pas la dose fractionnée saine à haut débit maximale (Dhh0ij), et
• la dose saine totale (DhTi) déposée dans lesdits tissus sains à la fin des N fractions ne dépasse pas la dose saine conventionnelle maximale (Dch0Ti),
dans lequel une cellule saine est située en aval de la surface périphérique quand un faisceau de particules chargées traverse ladite cellule saine uniquement après être sorti de la surface périphérique.

7. Système de planification de traitement selon l'une quelconque des revendications précédentes, dans lequel une ou plusieurs des fractions du plan comportent les étapes suivantes réalisées dans n'importe quel ordre séquentiel,
• une première étape de délivrance d'une dose conventionnelle à un débit de dépôt de dose conventionnel (CDR) avec un premier faisceau de particules chargées ou groupe de faisceaux de particules chargées parallèles à une première direction formés chacun par différents mini-faisceaux, de telle sorte que
o une dose fractionnée cible conventionnelle (Dctj) est délivrée à chacune ou certaines de la ou des fractions à au moins 90 %, de préférence au moins 95 %, mieux encore au moins 99 % des cellules tumorales du tissu cible (3t), et
o une dose fractionnée saine conventionnelle (Dchi) inférieure à la dose fractionnée saine conventionnelle maximale (Dch0ij) est délivrée à chacune ou certaines de la ou des fractions aux tissus sains (3hi) entourant et inclus à l'intérieur de la surface périphérique (c.-à-d. Dchij < Dch0ij), et
• une deuxième étape, suivant la première étape, de délivrance d'une dose à haut débit à un débit de dépôt de dose ultrahaut (HDR) avec un deuxième faisceau de particules chargées ou groupe de faisceaux de particules chargées parallèles à une deuxième direction identique ou différente de la première direction, de telle sorte que
o une dose fractionnée cible à haut débit (Dhtj) est délivrée à chacune ou certaines de la ou des fractions au tissu cible (3t) de telle sorte que 100 % des cellules tumorales du tissu cible (3t) ont reçu une dose fractionnée à un débit conventionnel et/ou ultrahaut (CDR, HDR), et
o une dose fractionnée saine à haut débit (Dhhij) est délivrée à chacune ou certaines de la ou des fractions aux tissus sains (3hi) entourant et à l'intérieur de la surface périphérique, de telle sorte que la dose fractionnée saine équivalente (Dmhij = Dchij + α Dhhij) délivrée à la fois à un débit conventionnel et ultrahaut (CDR, HDR) à toutes les cellules saines ne dépasse pas la dose fractionnée saine conventionnelle maximale (Dmh0ij) (c.-à-d. Dmhij ≤ Dch0ij).

8. Système de planification de traitement selon la revendication 7 précédente, dans lequel le deuxième faisceau de particules chargées ou groupe de faisceaux de particules chargées ne chevauche pas le premier faisceau de particules chargées ou groupe de faisceaux de particules chargées dans n'importe quel tissu sain (3hi) entourant ou à l'intérieur de la surface périphérique.

9. Système de planification de traitement selon l'une quelconque des revendications 1 à 4, dans lequel le plan comporte un seul faisceau de particules chargées (PB) ou un groupe de faisceaux parallèles à une première direction formés par des mini-faisceaux, de telle sorte que
• une dose fractionnée cible conventionnelle (Dctj) est délivrée à au moins 90 %, de préférence au moins 95 %, mieux encore au moins 99 % des cellules tumorales du tissu cible (3t),
• une dose fractionnée saine conventionnelle (Dchij) inférieure à la dose fractionnée saine conventionnelle maximale (Dch0ij) est délivrée aux tissus sains (3hi) entourant et inclus à l'intérieur de la surface périphérique (c.-à-d. Dchij < Dch0ij),
• une dose fractionnée cible à haut débit (Dhtj) est délivrée à chacune ou certaines de la ou des fractions au tissu cible (3t) de telle sorte que 100 % des cellules tumorales du tissu cible (3t) ont reçu une dose fractionnée à un débit conventionnel et/ou ultrahaut (CDR, HDR), et
• une dose fractionnée saine à haut débit (Dhhij) est délivrée à chacune ou certaines de la ou des fractions aux tissus sains (3hi) entourant et à l'intérieur de la surface périphérique, de telle sorte que la dose fractionnée saine équivalente (Dmhij = Dchij + α Dhhij) délivrée à la fois à un débit conventionnel et ultrahaut (CDR, HDR) à toutes les cellules saines ne dépasse pas la dose fractionnée saine conventionnelle maximale (Dch0ij) (c.-à-d. Dmhij ≤ Dch0ij).

10. Système de planification de traitement selon l'une quelconque des revendications 2 à 9 précédentes, dans lequel la surface périphérique comprend un volume incertain définissant une zone incertaine (3z), définie comme une zone comprenant à la fois des cellules tumorales et saines mélangées dans différentes proportions, et dans lequel le TPS comprend la délivrance à une totalité du volume incertain à la fin des N fractions d'une dose saine totale (DhTi), de telle sorte que
• la dose saine totale (DhTi = DchTi + DhhTi) est supérieure à la dose cible minimale (DmtT0), et inférieure à la dose saine à haut débit maximale (DhhT0i) (c.-à-d. DmtT0 < DhTi < DhhT0i), et
• une dose saine équivalente totale (DmhTi = DchTi + α DhhTi) délivrée à la fin des N fractions est inférieure à la dose saine conventionnelle maximale (DchT0i) du tissu sain (3hi) présent dans la zone incertaine (c.-à-d. DmhTi < DchT0i), et
• une dose fractionnée saine équivalente (Dmhij = Dchij + α Dhhij) délivrée à chaque fraction j est inférieure à la dose fractionnée saine conventionnelle maximale (DchT0ij) du tissu sain (3hi) présent dans la zone incertaine (c.-à-d. DmhTi < DchT0i).

11. Système de planification de traitement selon l'une quelconque des revendications précédentes, dans lequel la dose saine conventionnelle maximale (DchT0i) à la fin des N fractions est définie comme suit pour une sélection de tissus sains (3hi),
| Tissu sain (3hi) | DchT0i (Gy) |
|---|---|
| cerveau | 60 |
| coeur | 50 |
| larynx | 80 |
| foie | 40 |
| poumon | 25 |
| estomac | 65 |
| cristallin | 18 |

12. Système de planification de traitement selon la revendication précédente, dans lequel la dose saine à haut débit maximale (DhhT0i) à la fin des N fractions est définie comme suit pour une sélection de tissus sains (3hi),
| Tissu sain (3hi) | DhhT0i (Gy) |
|---|---|
| cerveau | 90 |
| coeur | 75 |
| larynx | 120 |
| foie | 60 |
| poumon | 38 |
| estomac | 98 |
| cristallin | 27 |

13. Système de planification de traitement selon l'une quelconque des revendications précédentes, dans lequel le faisceau de particules chargées est un faisceau de protons délivré dans un mode de diffusion simple ou de double diffusion, ou par balayage de faisceau filiforme (PBS) .
